(19) Europäisches Patentamt
European Patent Office
Office européen des brevets

(11) Publication number: **0 097 023**
**A2**

# (12) EUROPEAN PATENT APPLICATION

(21) Application number: **83303321.0**

(22) Date of filing: **08.06.83**

(51) Int. Cl.³: **C 07 C 177/00**, A 61 K 31/557, C 08 B 37/16

(30) Priority: **10.06.82 JP 98414/82**

(43) Date of publication of application: **28.12.83**
**Bulletin 83/52**

(84) Designated Contracting States: **AT BE CH DE FR GB IT LI LU NL SE**

(71) Applicant: **ONO PHARMACEUTICAL CO., LTD., No. 14, Doshomachi 2-chome Higashi-ku, Osaka-shi Osaka (JP)**

(72) Inventor: **Wakatsuka, Hiroshisa, 3-3-708, Miyano-cho, Takatsuki-shi Osaka (JP)**
Inventor: **Yamato, Takashi, 15-9-3-303, Tsukahara 1-chome, Takatsuki-shi Osaka (JP)**
Inventor: **Hashimoto, Shinsuke, 2-6-905, Shirakawa, 3-chome, Ibaraki-shi Osaka (JP)**

(74) Representative: **Bentham, Stephen et al, J.A. Kemp & Co. 14 South Square Gray's Inn, London WC1R 5EU (GB)**

(54) **Prostaglandin D derivatives, process for their preparation and compositions containing them.**

(57) Prostaglandin D derivatives of the formula:

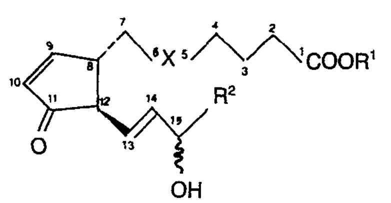

(I)

wherein $R^1$ is hydrogen or alkyl, $R^2$ is a group of the formula:

(i) 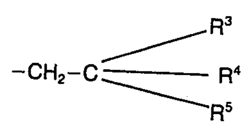 or

(ii) $-R^6-R^7$

wherein $R^3$ is alkyl, $R^4$ is hydrogen or alkyl, $R^5$ is alkyl $R^6$ is a single bond or alkylene, $R^7$ is cycloalkyl, X is ethylene or cis-vinylene, the double bond between $C_9-C_{10}$ is Z, and the double bond between $C_{13}-C_{14}$ is E, or of the formula:

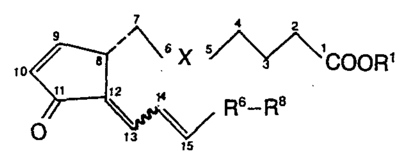

(II)

wherein $R^1$, $R^6$ and X are as hereinbefore defined, $R^8$ is alkyl, cycloalkyl, phenyl or phenoxy, the double bonds between $C_{12}-C_{13}$ and between $C_{14}-C_{15}$ are E, Z or a mixture thereof, and the double bond between $C_9-C_{10}$ is Z, provided that when $R^6$ represents a single bond, $R^8$ does not represent a substituted or unsubstituted phenoxy group, and cyclodextrin clathrates and non-toxic salts thereof, possess anti-tumour activity.

EP 0 097 023 A2

DESCRIPTION

"PROSTAGLANDIN D DERIVATIVES, PROCESS FOR THEIR PREPARATION AND COMPOSITIONS CONTAINING THEM"

The present invention relates to novel prostaglandin D derivatives, to processes for their production and pharmaceutical compositions containing them.

Prostaglandins are derivatives of prostanoic acid having the following structure:

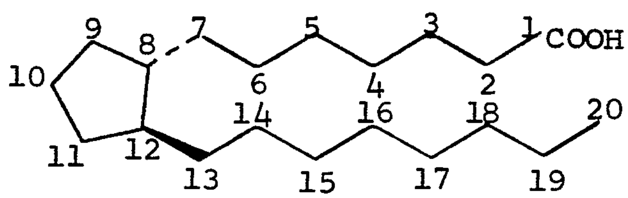

Various types of prostaglandins are known, and their types depend on the structure of the alicyclic ring and the substituents. For example, the alicyclic rings of prostaglandin F (PGF), E (PGE) and D (PGD) have the following structures, respectively:

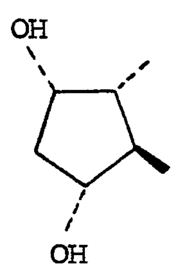

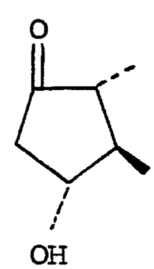

and

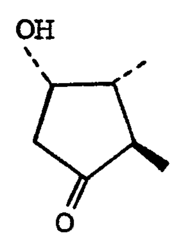

In the above structural formulae or in the other structural formulae in this specification, according to the generally accepted nomenclature, the broken line indicates that the substituent attached thereto is behind the ring plane, i.e. is of the α-configuration, the bold line ◄ indicates that the substituent attached thereto is in front of the ring plane, i.e. is of the β-configuration, and the wavy line ∿ indicates that the substituent attached thereto is of the α-configuration or the β-configuration or a mixture thereof.

These compounds are sub-classified according to the positions of the double bonds in the side chains attached to the alicyclic ring at the 8-position and the 12-position. The PG-1 compound has a _trans_ double bond (_trans_- $\Delta^{13}$) between $C_{13}$ - $C_{14}$ and the PG-2 compound has a _cis_ double bond between $C_5$ - $C_6$ and a _trans_ double bond between $C_{13}$ - $C_{14}$ (_cis_- $\Delta^5$, _trans_- $\Delta^{13}$). For example, prostaglandin $D_1$ ($PGD_1$) and prostaglandin $D_2$ ($PGD_2$) may be expressed by the following structural formulae respectively:

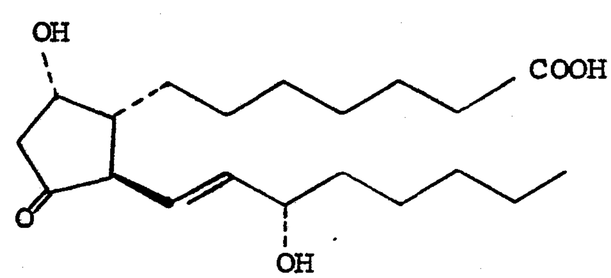

($PGD_1$)

and

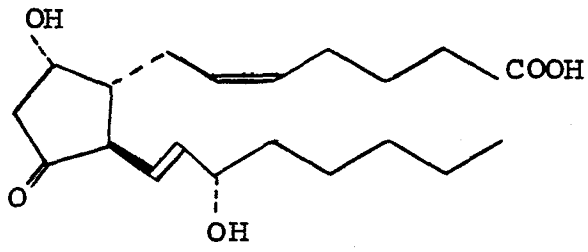

(PGD$_2$)

Further, when one or more methylene groups are removed from the aliphatic group attached at the 12-position of the alicyclic ring of a prostaglandin, said compound is known as a nor-prostaglandin according to the general rule of the organic nomenclature, and the number of the removed methylene groups is indicated by adding di-, tri- etc. before the prefix "nor".

The prostaglandins generally have pharmacological properties. For example, they exert various effects, including the stimulation of contraction of smooth muscles, a hypotensive effect, a diuretic effect, a bronchial dilation effect, the inhibition of lypolysis, the inhibition of platelet aggregation and the inhibition of gastric acid secretion. Therefore, they are useful in treatments of hypertension, thrombosis, asthma and gastric and intestinal ulcers, in the induction of labor and abortion in pregnant mammals, in the prevention of arteriosclerosis and also as diuretics. They are liposoluble substances present in extremely small quantities in the tissues which secrete prostaglandins in vivo in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been discovered that novel compounds in which the hydroxy group attached at the 9-position and the hydrogen atom attached at the 10-position of $PGD_1$ and $PGD_2$ have been removed to introduce a _cis_ double bond between $C_9$ - $C_{10}$ (9-deoxy—$\Delta^9$-PGD analogues) and related novel compounds in which, in addition, the hydroxy group attached to the 15-position has been removed to introduce double bonds between the $C_{12}$ - $C_{13}$ and $C_{14}$ - $C_{15}$ positions have a surprisingly strong anti-tumour effect whereas they have no or very weak pharmacological properties possessed by the "natural" prostaglandins.

The fact that $PGD_2$ inhibits the proliferation of cancerized mast cells has already been made public [see Proceedings of the Japanese Cancer Association, the 40th Annual Meeting (published on September 5, 1981)]. However, the anti-tumour effect of compounds of the present invention which are structurally distinguished from $PGD_2$ has been found to be superior by several times as compared with that of $PGD_2$ whereas in other PG-like properties compounds of the present invention are several times less

active than $PGD_2$. The compounds of the invention tested therefore exert an anti-tumour effect which is selective in relation to the other effects, a particularly advantageous selective action compared with $PGD_2$.

Heretofore, there have been filed several patent applications relating to 9-deoxy-$\Delta^9$-PGD analogues. For example, there are disclosed 9-deoxy-$\Delta^9$-$PGD_2$ and alkyl esters thereof in the specification of the United States Patent No. 3954844 (Derwent No. 37947X); compounds wherein various substituents have been introduced into the aliphatic group attached to the 12-position are described in the specification of the United States Patent No. 4016184 (Derwent No. 24680Y); and further compounds in which the carboxyl group at the 1-position of the 9-deoxy-$\Delta^9$-PGD analogues have been replaced by a hydroxymethyl group are described in the specifications of the United States Patent Nos. 4028419 (Derwent No. 43349Y), 4055602 (Derwent No. 48794Y) and 4032576 (Derwent No. 48793Y).

However, none of the above applications discloses the compounds of the present invention.

Compounds of the invention which have double bonds between $C_{12}$ - $C_{13}$ and $C_{14}$ - $C_{15}$ are also novel: compounds having such a structure have not heretofore been discovered.

Furthermore, in addition to their structural

differences from known PGD compounds, the novel compounds of the present invention have an effect which has not hitherto been found in the known 9-deoxy-$\Delta^9$-PGD analogues that is, an anti-tumour effect. The above-described United States Patent specifications describe only the pharmacological properties known with the natural prostaglandins and none of the applications refers to an anti-tumour effect.

Accordingly, the present invention relates to prostaglandin D derivatives of the general formula:

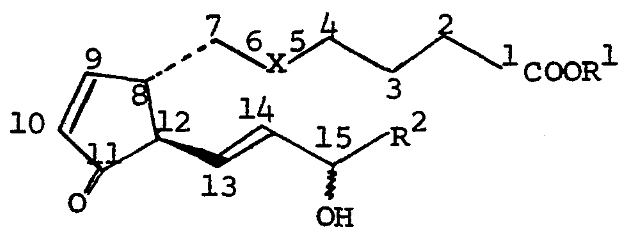

(I)

wherein $R^1$ represents a hydrogen atom or a straight-chain or branched-chain alkyl group of 1 - 12 carbon atoms, $R^2$ represents a group of the general formula:

(i) 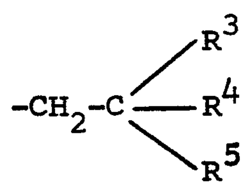 or

(ii) $-R^6-R^7$

wherein $R^3$ represents a straight-chain or branched-chain alkyl group of 1 - 4 carbon atoms, $R^4$ represents a hydrogen

atom or a straight-chain or branched-chain alkyl group of 1 - 4 carbon atoms, $R^5$ represents a straight-chain or branched-chain alkyl group of 1 - 10 carbon atoms, $R^6$ represents a single bond or a straight-chain or branched-chain alkylene group of 1 - 5 carbon atoms, and $R^7$ represents a cycloalkyl group of 4 - 7 carbon atoms either unsubstituted or substituted by at least one straight-chain or branched-chain alkyl group of 1 - 8 carbon atoms, X represents an ethylene group ($-CH_2CH_2-$) or a cis-vinylene group (cis-CH=CH-), the double bond between $C_9$ - $C_{10}$ is Z, and the double bond between $C_{13}$ - $C_{14}$ is E, and of the general formula:

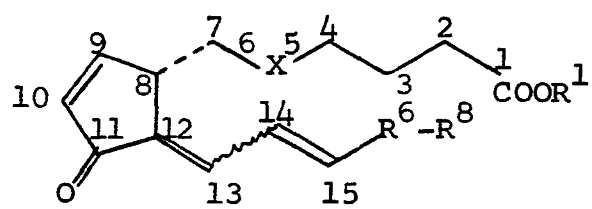

(II)

wherein $R^1$, $R^6$ and X are as hereinbefore defined, $R^8$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms or represents a phenyl group or phenoxy group either unsubstituted or substituted by at least one halogen atom, trifluoromethyl group or straight-chain or branched-chain alkyl group of 1 - 4 carbon atoms, the

double bonds between $C_{12}$ - $C_{13}$ and between $C_{14}$ - $C_{15}$ which may be in the same or different configurations, are E, Z or a mixture thereof (i.e. EZ), and the double bond between $C_9$ - $C_{10}$ is Z, provided that when $R^6$ represents a single bond, $R^8$ does not represent a substituted or unsubstituted phenoxy group, and cyclodextrin clathrates of compounds of general formulae (I) and (II), and non-toxic salts of acids of general formulae (I) and (II) wherein $R^1$ represents a hydrogen atom.

In the compounds of the general formula (I), there are at least three asymmetric carbons (that is, carbon atoms at the 8-, 12- and 15-positions), and in the compounds of the general formula (II), there is at least one asymmetric carbon atom (the carbon atom at the 8-position). Further asymmetric carbon atoms may occur in the groups represented by $R^1$, $R^2$, $R^6$ and $R^8$. The presence of asymmetric carbon atoms leads to the existence of isomerism. It is to be understood that all isomers and mixtures thereof arising from the presence of asymmetric carbon atoms are to be considered within the scope of formulae I and II.

In the general formulae (I) and (II), as the

alkyl group of 1 - 12 carbon atoms represented by $R^1$, there may be mentioned methyl, ethyl, propyl, butyl, pentyl, hexyl, heptyl, octyl, nonyl, decyl, undecyl, dodecyl groups and isomers thereof; $R^1$ preferably represents a hydrogen atom or an alkyl group of 1 - 4 carbon atoms, and more especially a hydrogen atom or a methyl or ethyl group.

In the general formula (I), as the alkyl group of 1 - 4 carbon atoms represented by $R^3$ and $R^4$, there may be mentioned methyl, ethyl, propyl, butyl and isomers thereof. $R^3$ is preferably a methyl or ethyl group (especially methyl), and $R^4$ is preferably a hydrogen atom.

In the general formula (I), as the alkyl group of 1 - 10 carbon atoms represented by $R^5$, there may be mentioned methyl, ethyl, propyl, butyl, pentyl, hexyl, heptyl, octyl, nonyl, decyl and isomers thereof. $R^5$ is preferably a propyl, butyl or pentyl group.

In the general formulae (I) and (II), as the alkylene group of 1 - 5 carbon atoms represented by $R^6$ there may be mentioned methylene, ethylene, trimethylene, tetramethylene, pentamethylene and isomers thereof. $R^6$ preferably represents a single bond or a methylene or ethylene group [in particular a single bond in formula (I) and a single bond and a methylene group in formula (II)].

In the general formulae (I) and (II), as the substituted or unsubstituted cycloalkyl group represented

by $R^7$ and $R^8$ respectively, there may be mentioned cyclobutyl, cyclopentyl, cyclohexyl, cycloheptyl and cyclobutyl, cyclopentyl, cyclohexyl and cycloheptyl groups substituted by one or more methyl, ethyl, propyl, butyl, pentyl, hexyl, heptyl or octyl groups; cyclobutyl, cyclopentyl or cyclohexyl groups either unsubstituted or substituted by one methyl, ethyl, propyl or butyl group are preferred; alkyl-substituted cyclopentyl is especially preferred.

In the general formula (II), as the alkyl group of 1 - 8 carbon atoms represented by $R^8$, there may be mentioned methyl, ethyl, propyl, butyl, pentyl, hexyl, heptyl, octyl and isomers thereof; butyl,pentyl or hexyl groups either unsubstituted or substituted by one or two methyl or ethyl groups are preferred; pentyl and hexyl unsubstituted or substituted by one or two methyl groups are especially preferred.

In the general formula (II), as the substituted or unsubstituted phenyl or phenoxy group represented by $R^8$, there may be mentioned phenyl and phenoxy groups, and phenyl and phenoxy groups substituted by one or more atoms or groups selected from fluorine and chlorine atoms, and trifluoromethyl, methyl, ethyl, propyl or butyl groups; phenyl or phenoxy groups either unsubstituted or substituted by one chlorine atom, trifluoromethyl group, methyl group or ethyl group are preferred; chlorine is a

preferred substituent.

In the general formula (I), as a preferred $R^2$, there may be mentioned 2-methylpentyl, 2-ethylpentyl, 2-methylhexyl, 2-ethylhexyl, 2-methylheptyl, and 2-ethylheptyl; and also cyclobutyl, 1-propylcyclobutyl, 1-butylcyclobutyl 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl and 4-butylcyclohexyl groups; 2-methylhexyl and 3-propylcyclopentyl are especially preferred.

In the general formula (II), as a preferred $R^6$ - $R^8$, there may be mentioned butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, and 2-ethylheptyl; cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, and 4-butylcyclohexyl; and also benzyl, 2-phenylethyl, 4-methylbenzyl, 4-ethylbenzyl, phenoxymethyl, 2-phenoxyethyl, 3-chlorophenoxymethyl, 4-chlorophenoxymethyl,

3-trifluoromethylphenoxymethyl, 4-trifluoromethylphenoxymethyl, 4-methylphenoxymethyl and 4-ethylphenoxymethyl; pentyl, 1,1-dimethylpentyl, 2-methylhexyl, 3-propylcyclopentyl, benzyl and 3-chlorophenoxymethyl are especially preferred.

Further, in the general formulae (I) and (II), X is preferably a <u>cis</u>-vinylene group, and in the general formula (I), the preferred configuration of the hydroxyl group attached to the carbon atom at the 15-position is the α-configuration.

According to a feature of the present invention, the prostaglandin derivatives of the general formula (II) wherein the various symbols are as hereinbefore defined may be prepared by reacting a compound of the general formula:

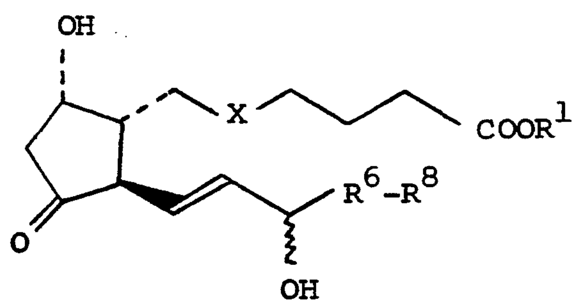

(III),

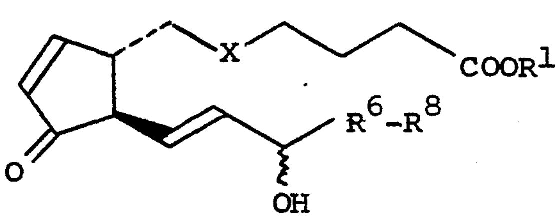

(IV),

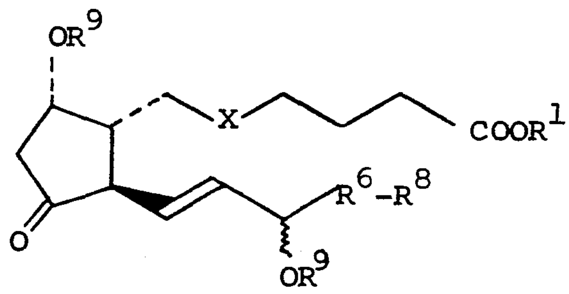

(V)

or

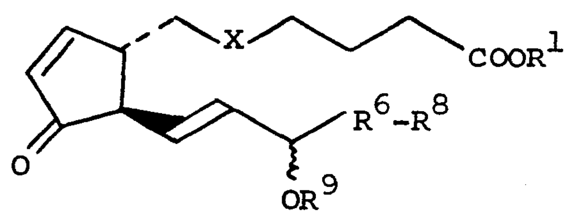

(VI)

wherein X, $R^1$, $R^6$ and $R^8$ are as hereinbefore defined and $R^9$ represents a tetrahydropyran-2-yl group, tetrahydrofuran-2-yl group either unsubstituted or substituted by at least one alkyl group, or a 1-ethoxyethyl group, preferably, a tetrahydropyran-2-yl group, with an aqueous acid, preferably in an inert organic solvent, for example, methylene chloride, chloroform, carbon tetrachloride, diethyl ether, N,N-dimethylformamide, tetrahydrofuran or a mixture of two or more such solvents, using as the aqueous acid, for example, an aqueous solution of an inorganic acid, eg hydrochloric acid, hydrobromic acid, sulfuric acid or phosphoric acid or an organic acid, e.g.

acetic acid, propionic acid or oxalic acid, at the reflux temperature of the solvent. The reaction is preferably carried out in tetrahydrofuran using 1 N hydrochloric acid at the reflux temperature of the reaction mixture.

The prostaglandin derivatives of the general formulae (III) and (IV) wherein the various symbols are as hereinbefore defined may be prepared by hydrolyzing compounds of the general formulae (V) and (VI), respectively, under acidic conditions. This hydrolysis may be conducted, for example:

(1) in an aqueous solution of an organic acid such as acetic acid, propionic acid, oxalic acid or p-toluenesulfonic acid or an aqueous solution of an inorganic acid such as hydrochloric acid, sulfuric acid or phosphoric acid, suitably in the presence of a water-miscible organic solvent, for example, a lower alkanol such as methanol or ethanol (preferably methanol) or an ether such as 1,2-dimethoxyethane, dioxan or tetrahydrofuran (preferably tetrahydrofuran) at a temperature of from room temperature to 80°C, or

(2) in an anhydrous lower alkanol such as methanol or ethanol in the presence of an organic acid such as p-toluenesulfonic acid or trifluoroacetic acid at a temperature of 10 - 45°C.

The hydrolysis is preferably conducted using a mixture of dilute hydrochloric acid and tetrahydrofuran, a

mixture of dilute hydrochloric acid and methanol, a mixture of acetic acid, water and tetrahydrofuran, a mixture of phosphoric acid, water and tetrahydrofuran, or a mixture of p-toluenesulfonic acid and anhydrous methanol.

The prostaglandin derivatives of the general formula (IV) wherein the various symbols are as hereinbefore defined may also be prepared by subjecting a compound of the general formula (III) to a mild dehydration reaction, for example, by reaction with a tris hydrochloric acid buffer solution at a temperature of 30 - 40°C.

The prostaglandin derivatives of the general formula (I) wherein the various symbols are as hereinbefore defined may be prepared by subjecting a compound of the general formula (VI) wherein the grouping $-R^6-R^8$ in the general formula (VI) represents $R^2$, i.e. a compound of the general formula:

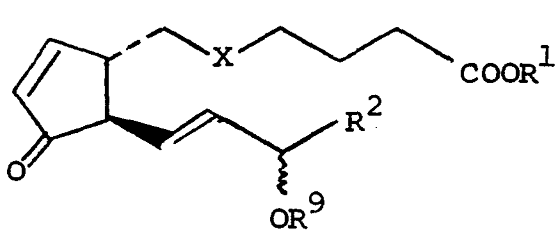

(VIa)

(wherein the various symbols are as hereinbefore defined) to hydrolysis as hereinbefore described for the conversion of compounds of general formulae (V) and (VI) to compounds of general formulae (III) and (IV), or by subjecting a compound of the general formula (III) wherein $-R^6-R^8$ in the general formula (III) represents $R^2$,

i.e. a compound of the general formula:

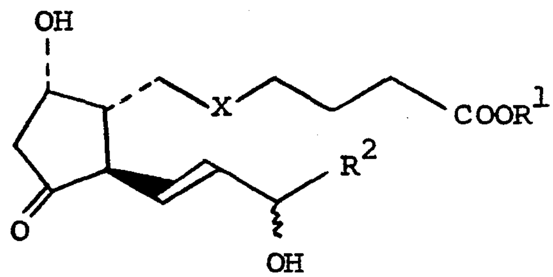

(IIIa)

(wherein the various symbols are as hereinbefore defined) to a mild dehydration reaction as hereinbefore described for the conversion of compounds of general formula (III) to compounds of general formula (IV).

According to a further feature of the present invention, the esters of the general formulae (I), (II), (III) and (IV) wherein $R^1$ represents a straight-chain or branched-chain alkyl group of 1 - 12 carbon atoms and the other symbols are as hereinbefore defined may also be produced by esterifying the acids of the general formulae (I), (II), (III) and (IV), respectively, wherein $R^1$ represents a hydrogen atom, and the other symbols are as hereinbefore defined, by known methods.

By the expression "known methods" as used in this specification and the accompanying claims is meant methods heretofore used or described in the literature.

The esterification reaction may, for example, be carried out by:

(1) a process employing a diazoalkane, or

(2) a process employing an N,N-dimethylformamide - dialkyl

acetal, or

(3) a process employing a dicyclohexylcarbodiimide and an appropriate alcohol as described in Japanese Patent Specification No. 762305, or

(4) a process employing a pivaloyl halide and an appropriate alcohol as described in Japanese Patent Specification No. 756972, or

(5) a process employing an arylsulfonyl halide or an alkylsulfonyl halide and an appropriate alcohol as described in Japanese Patent Specification No. 759351.

The process employing a diazoalkane may be conducted by reaction with an appropriate diazoalkane in an inert organic solvent, for example, diethyl ether, ethyl acetate, methylene chloride, acetone or a mixture of two or more such solvents, at from -10°C to room temperature, preferably at 0°C.

The process employing an N,N-dimethylformamidedialkyl acetal may be conducted by reaction with an N,N-dimethylformamide - dialkyl acetal, for example, N,N-dimethylformamide-dimethyl acetal, in anhydrous benzene at from 0°C to room temperature.

The process employing a dicyclohexylcarbodiimide may be conducted by reacting an appropriate acid and an appropriate alcohol in an inert organic solvent, for example, a halogenated hydrocarbon such as chloroform or methylene chloride, in the presence of a base such as

pyridine or picoline or, preferably, 4-dimethylaminopyridine, at a temperature of 0°C to room temperature.

The process employing a pivaloyl halide, or an arylsulfonyl halide or an alkylsulfonyl halide may be conducted by adding a tertiary amine such as triethylamine, or pyridine, and a pivaloyl halide, for example, pivaloyl chloride, or an arylsulfonyl halide, for example, benzenesulfonyl chloride or p-toluenesulfonyl chloride or an alkylsulfonyl halide, for example, methanesulfonyl chloride or ethanesulfonyl chloride either in an inert organic solvent, for example, halogenated hydrocarbons such as chloroform or methylene chloride or diethyl ether or in the absence of a solvent to form a mixed acid anhydride, and subsequently adding the appropriate alcohol and reacting at a temperature of 0°C to room temperature.

Starting materials of the general formulae (V) and (VI) may be prepared by the process described in the specifications of the United States Patent Nos. 3878239 (Derwent No. 76109U) and 4016184 (Derwent No. 24680Y), and they may also be produced by the sequence of reaction steps illustrated in the following Scheme A. In Scheme A, $R^{1a}$ represents a straight-chain or branched-chain alkyl group of 1 - 4 carbon atoms, $R^{10}$ represents a straight-chain or branched-chain alkyl group of 1 - 4 carbon atoms or a phenyl group, preferably a n-butyl group or a phenyl group, $R^{11}$ represents a straight-chain or branched-chain acyl group of 2—5 carbon atoms or a benzoyl group, preferably a benzoyl

group, or a tri-substituted silyl group such as a trimethylsilyl, triethylsilyl, tributylsilyl, tert-butyldimethylsilyl, tribenzylsilyl or triphenylsilyl group, preferably a tert-butyldimethylsilyl group, $R^{12}$ represents an alkylsulfonyl group, or a substituted or unsubstituted arylsulfonyl group, preferably a mesyl group or a tosyl group, and the other symbols are as hereinbefore defined.

Scheme A

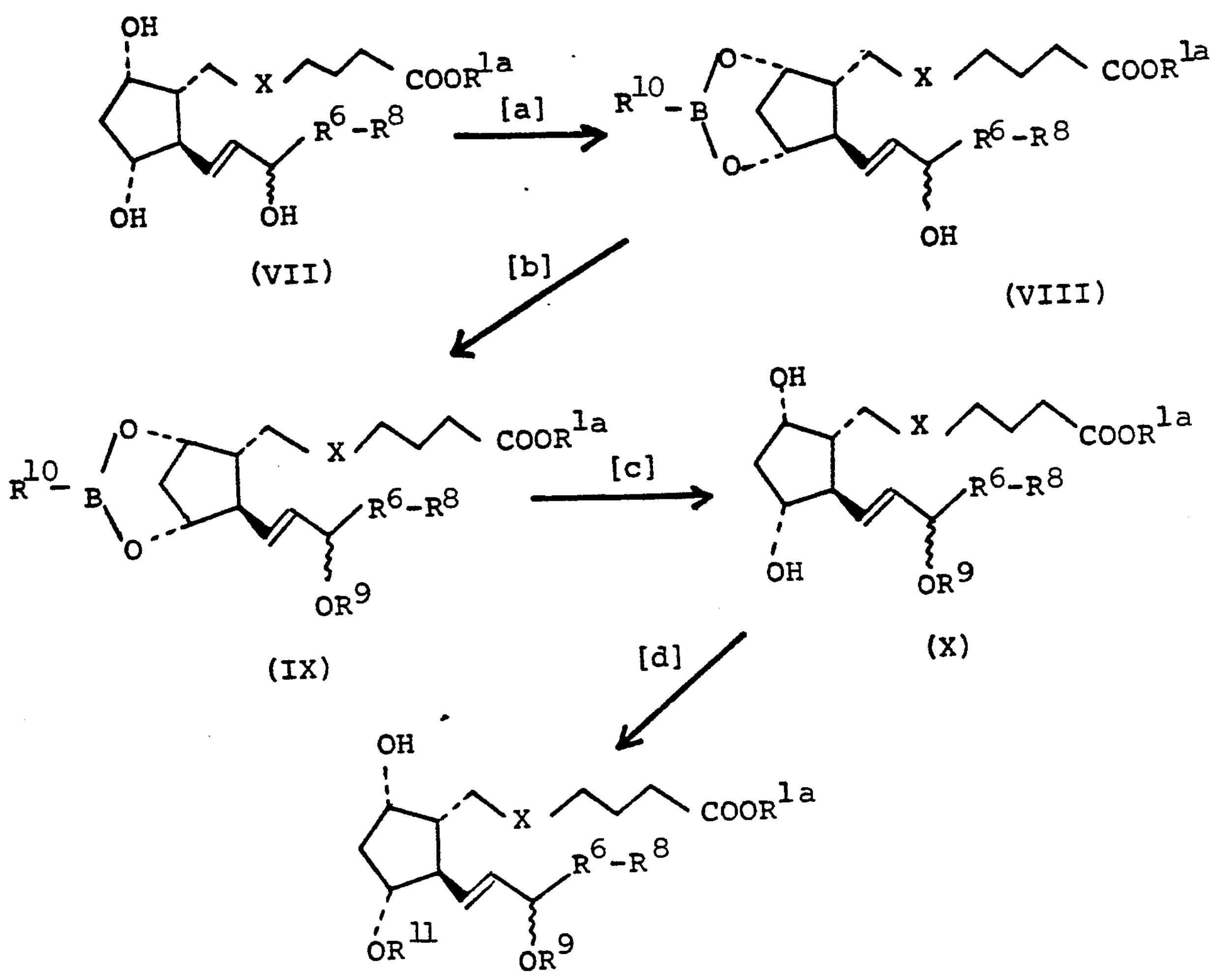


(VII) (VIII) (IX) (X) (XI)

Scheme A (Continued)

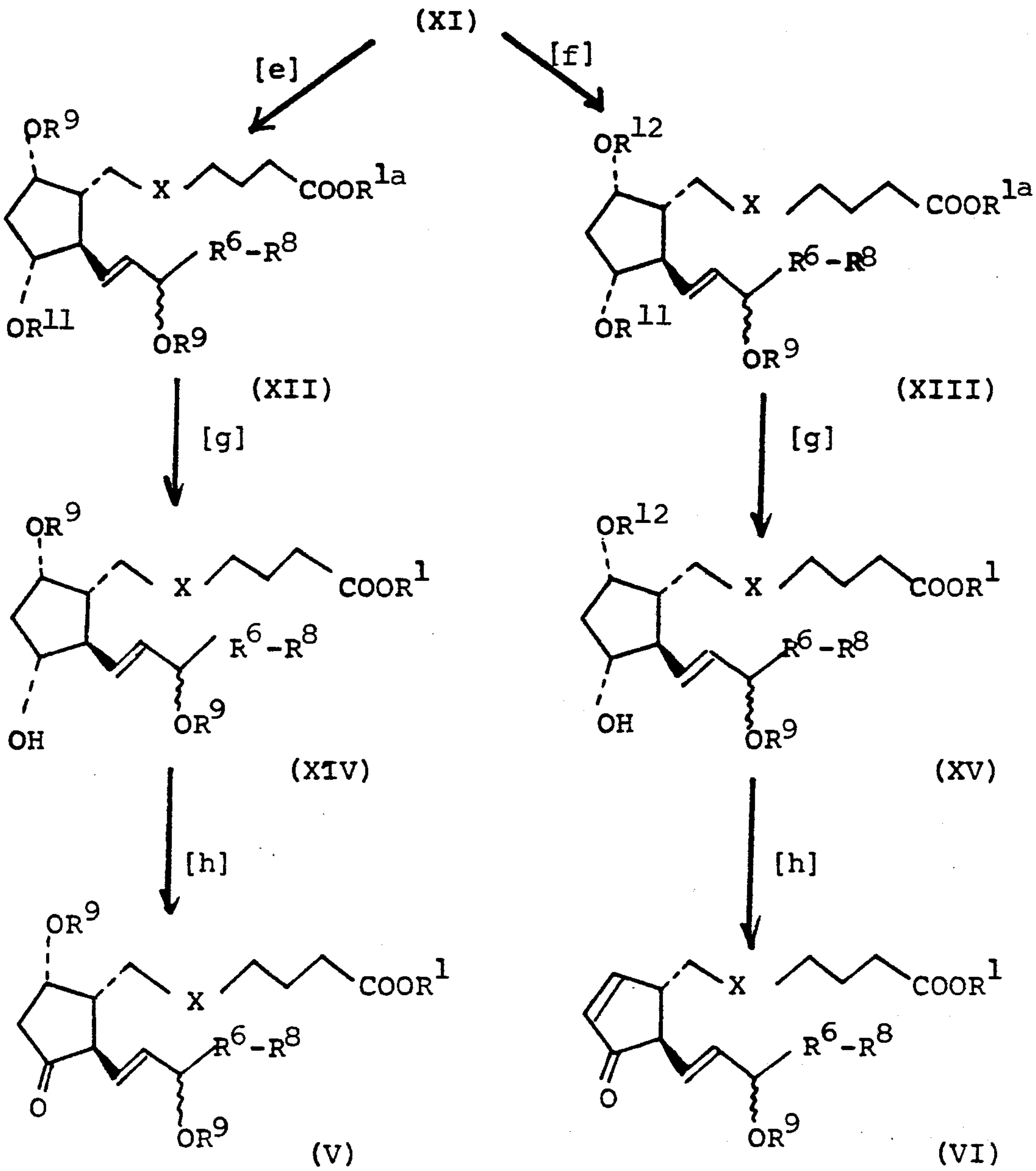


(XI)
(XII)
(XIII)
(XIV)
(XV)
(V)
(VI)

All of the reaction steps in Scheme A may be conducted by known methods. For example, the step [a] may be conducted by reacting with a boric acid corresponding to $R^{10}$, for example, phenylboric acid [$PhB(OH)_2$] or butylboric acid [$n-BuB(OH)_2$], in an inert organic solvent such as methylene chloride, chloroform, carbon tetrachloride or tetrahydrofuran in the presence of molecular sieves 3A or 4A at reflux temperature [see J. C. S. Chem. Comm., page 658 (1975) and Prostaglandins, vol. 9, page 109 (1975)].

The step [b] may be conducted using, eg 2,3-dihydropyran, 2,3-dihydrofuran or ethyl vinyl ether in an inert organic solvent, for example, methylene chloride, chloroform or diethyl ether, in the presence of a condensing agent, for example, p-toluenesulfonic acid, sulfuric acid or trifluoroacetic acid at a temperature of from room temperature to -30°C. Preferably, it is conducted using 2,3-dihydropyran in methylene chloride in the presence of pyridine p-toluenesulfonate or p-toluenesulfonic acid at room temperature.

The step [c] may be conducted by reacting with an aqueous hydrogen peroxide solution in an aqueous solution of an alkanol of 1 - 4 carbon atoms, for example, aqueous methanol or aqueous ethanol, at a temperature of not higher than 60°C, preferably at 45 - 50°C.

The products of steps [a], [b] and [c] may be used without purification, in subsequent steps.

The step [d] may be conducted by selectively acylating or silylating the hydroxy group attached to the 11-position of the compound of the general formula (X). Such acylation may be conducted using an appropriate acyl chloride or acid anhydride, for example, benzoyl chloride, in an inert organic solvent, for example, methylene chloride, or in the absence of a solvent in the presence of a tertiary amine such as pyridine or triethylamine at a temperature not higher than room temperature, preferably at -30 to -40°C. A suitable silylation procedure is described in detail in "Protective Groups in Organic Synthesis" published by John Wiley & Sons, Inc. (USA), page 39 - 50 (1981) [referred to as "reference A" hereafter], and may be conducted by reacting with a chlorosilane compound of the general formula: $Cl-R^{11a}$,wherein $R^{11a}$ represents a tri-substituted silyl group, in an inert organic solvent such as dimethylaminopyridine or dimethylformamide in the presence or a tertiary amine such as pyridine, imidazole or triethylamine at a temperature of from room temperature to 50°C.

The step [e] may be conducted as described for step [b].

The step [f] may be conducted by reacting with an alkylsulfonyl chloride such as mesyl chloride or an arylsulfonyl chloride such as tosyl chloride at a temperature of from -30°C to 50°C (i) in an inert organic solvent such as methylene chloride in the presence of a tertiary amine such as pyridine or triethylamine, or (ii) in pyridine.

The step [g] may be conducted by deacylation or desilylation. Such deacylation may be conducted by (1) using a hydroxide of an alkali metal such as lithium, sodium or potassium in an aqueous alkanol such as aqueous methanol or aqueous ethanol at a temperature of not lower than room temperature, preferably at 50 - 60°C, to give compounds of general formulae (XIV) and (XV) wherein $R^1$ represents a hydrogen atom, or (2) using anhydrous potassium carbonate in an anhydrous alkanol, such as anhydrous methanol, to give compounds of general formulae (XIV) and (XV) wherein $R^1$ represents an alkyl group of 1 to 4 carbon atoms. If desired, acids of the general formulae (XIV) and (XV) may be converted into the corresponding esters by the known esterification processes hereinbefore described. The desilylation above is described in detail in reference A and is preferably conducted by using tetrabutylammonium fluoride ($n\text{-}Bu_4N^+F^-$) in tetrahydrofuran at room temperature to give compounds of general formulae (XIV) and (XV) wherein $R^1$ represents an alkyl group of 1 - 4 carbon atoms.

If desired, esters of the general formulae (XIV) and (XV) may be converted into the corresponding acids by the method (1) described above for deacylation.

The step [h] is an oxidation reaction to convert the 11-hydroxy group in the compounds of general formulae (XIV) and (XV) into an oxo group and, in addition to eliminate the group $OR^{12}$ in the compound of general formula (XV) to from a double bond between $C_9$-$C_{10}$. Such an oxidation reaction is well known, and is described in detail in, for example,

(a) "Synthetic Organic Chemistry III, Organic Synthesis 1", pp. 176-206 (compiled by Tetsuji Kameya and published by Nankodo (Japan) on Aug. 1, 1976) or

(b) "Compendium of Organic Synthetic Methods", vol. 1, vol. 2, and vol. 3, section 48 or 168 [published by John Wiley & Sons, Inc. (USA) in 1971, 1974, and 1977, respectively].

The oxidation is preferably carried out under mild neutral conditions using, for example, dimethylsulphide-N-chlorosuccinimide complex, thioanisole-N-chlorosuccinimide complex, dimethylsulphide-chlorine complex, thioanisole-chlorine complex [see J. Amer. Chem. Soc., 94, 7586 (1972) with respect to these complexes], dicyclohexylcarbodiimide-dimethylsulphoxide complex [see J. Amer. Chem. Soc., 87, 5661 (1965)], pyridinium chlorochromate ($C_5H_5NHCrO_3Cl$) [see Tetrahedron Letters, 2647 (1975)], sulphuric anhydride-pyridine complex [see J. Amer. Chem. Soc., 89, 5505 (1967)], chromyl chloride [see. J. Amer. Chem. Soc., 97, 5929 (1975)], chromium trioxide-pyridine complex (for example, Collins' reagent), Jones' reagent or chromic acid solution (prepared from chromium trioxide, manganese sulfate, sulfuric acid and water), or oxalyl chloride and dimethylsulfoxide (i.e. Swern oxidation); suitably the Collins oxidation, Jones oxidation or Swern oxidation may be employed. The Collins oxidation may be conducted in a halogenated hydrocarbon such as chloroform, methylene chloride or carbon tetrachloride at a temperature of from room temperature to 0°C, preferably at 0°C. The Jones oxidation is generally

conducted at a temperature of not higher than room temperature. The Swern oxidation may be conducted by reaction in a halogenated hydrocarbon such as chloroform or methylene chloride at a temperature of from -50°C to -60°C, and then treatment with triethylamine.

In the sequence of the reaction steps illustrated by the above Scheme A, the compounds of the general formula (VII) employed as the starting material are known _per se_ as PGF derivatives, and may be produced by converting the $OR^9$ groups at the 11-position and the 15-position of a compound of the general formula:

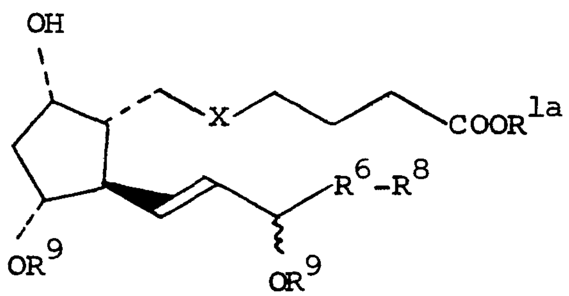

(XVI)

(wherein the various symbols are as hereinbefore defined) to hydroxy groups by the hydrolysis under acidic conditions hereinbefore described for the conversion of compounds of general formula (V) to those of general formula (III).

Compounds of general formula (XVI) can be obtained by the methods described in the following literature references and patent specifications, or obvious modifications thereof:-

(1) when the group $-R^6-R^8$ represents a pentyl group, as described in J. Amer. Chem. Soc., 91, 5675 (1969) or _ibid._, 92, 397 (1970);

(2) when the group $-R^6-R^8$ represents an alkyl group, as described in Japanese Patent Kokai Nos. 42675/72, 54068/73, 64073/73, 124048/74, 95250/75, 96543/75 and 101340/75, British Patent Specification Nos. 1398291, 1483240 and 1540427, United States Patent Specification No. 4024174, and Belgian Patent No. 850084;

(3) when $R^8$ of the group $-R^6-R^8$ represents a cycloalkyl group, as described in Japanese Patent Kokai Nos. 109353/74, 95250/75, 96543/75, 123647/75, 148339/75, 122040/76, 125256/76, 27753/77 and 25544/78, British Patent Specification Nos. 1464916, 1488141, 1483240, 1484210 and 1545213, United States Patent Specifications Nos. 3966792, 4034003, 4024174, 4045468 and 4087620, and Belgian Patent No. 844256;

(4) when $R^8$ of the group $-R^6-R^8$ represents a phenyl group or a phenoxy group, as described in Japanese Patent Kokai Nos. 95250/75, 96543/75, 59841/76, 101961/76 and 25745/77, British Patent Specifications Nos. 1483240 and 1521747, United States Patent Specification Nos. 4024174 and 4065632, and Belgian Patent No. 845348.

Cyclodextrin clathrates of the compounds of general formulæ (I) and (II) can be prepared by dissolving the cyclodextrin in water or a water-miscible organic solvent, and adding to the solution the prostaglandin

derivative in a water-miscible organic solvent. The mixture is then heated and the desired cyclodextrin clathrate is isolated from the resulting solution by concentrating the mixture under reduced pressure, or by cooling and separating the product by filtration or decantation. The ratio of organic solvent to water may be varied according to the solubilities of the starting materials and products. Preferably, the temperature is not allowed to exceed 70°C during preparation of the cyclodextrin clathrate. α-, β- or γ-Cyclodextrin, or mixtures thereof, may be used to prepare the cyclodextrin clathrates. Conversion into cyclodextrin clathrates serves to increase the stability of the prostaglandin derivatives of the general formulae (I) and (II).

The prostaglandin derivatives of general formula (I) or (II) wherein $R^1$ represents a hydrogen atom may, if desired, be converted by known methods into salts. Preferably the salts are non-toxic salts. By the term "non-toxic salts", as used in this specification, is meant salts the cations of which are relatively innocuous to the animal organism when used in therapeutic doses so that the beneficial pharmacological properties of the compounds of general formula (I) or (II) are not vitiated by side-effects ascribable to those cations. Preferably the salts are water-soluble. Suitable non-toxic salts include the alkali metal, e.g.

sodium or potassium, salts, the alkaline earth metal, e.g. calcium or magnesium, salts and ammonium salts, and pharmaceutically acceptable, (i.e. non-toxic) amine salts. Amines suitable for forming such salts with a carboxylic acid are well known and include, for example, amines derived in theory by the replacement of one or more of the hydrogen atoms of ammonia by groups, which may be the same or different when more than one hydrogen atom is replaced, selected from, for example, alkyl groups containing from 1 to 6 carbon atoms and hydroxyalkyl groups containing 2 or 3 carbon atoms. Suitable non-toxic amine salts are, e.g., tetraalkylammonium, such as tetramethylammonium, salts, and other organic amine salts such as methylamine salts, ethylamine salts, isopropylamine salts, <u>tert</u>-butylamine salts, dimethylamine salts, cyclopentylamine salts, benzylamine salts, phenethylamine salts, piperidine salts, monoethanolamine salts, diethanolamine salts, lysine salts or arginine salts.

Salts may be prepared from the acids of general formula (I) or (II) wherein $R^1$ represents a hydrogen atom, by known methods, for example by reaction of stoichiometric quantities of an acid of general formula (I) or (II) and the appropriate base, e.g. an alkali metal or alkaline earth metal hydroxide or carbonate, ammonium hydroxide, ammonia or an organic amine, in a suitable solvent. The salts

may be isolated by lyophilisation of the solution or, if sufficiently insoluble in the reaction medium, by filtration, if necessary, after removal of part of the solvent.

The prostaglandin D derivatives of the general formula (I) or (II), and cyclodextrin clathrates thereof, and non-toxic salts of the acids when $R^1$ in the general formulae (I) and (II) represents a hydrogen atom either do not show the pharmacological effects typical of other prostaglandins, for example, hypotensive activity, inhibition of platelet aggregation, stimulation of uterine muscles and the production of diarrhoea or these effects are very weak. In contrast the anti-tumour effect of the derivatives of general formula (I) and (II) is extremely strong and moreover their toxicity is extremely low. They may therefore be employed as very effective anti-tumour agents in the prevention or therapy of leukemia and solid cancer, and in treatment to produce remission thereof.

In a laboratory, <u>in vitro</u> test on the inhibition of proliferation of human myelogenous leukemic cells (HL-60) (the experimental method being described in detail below), the compound of the present invention, (5Z,9Z,14EZ)-11-oxoprosta-5,9,12,14-tetraenoic acid [produced in Example 2 (d)] inhibited the proliferation of the leukemic cells by 93.2% at a concentration of 5 μg/ml. In contrast, $PGD_2$ (which is known to exert an anti-tumour effect) inhibited

the proliferation of the leukemic cells by only 55.6% at a concentration of 5 μg/ml in the same test.

The test on the inhibition of the proliferation of human myelogenous leukemic cells was conducted by a well known method. The human myelogenous leukemic cells (HL-60) were added to an RPMI culture solution containing 10% bovine fetal serum, the number of cells in the culture solution was adjusted to $1 \times 10^5$ cells/ml, a solution in ethanol of the compound under test was added to give a concentration of 5μg/ml, and the mixture was subjected to stationary culture at 37°C for 4 days. As a control, a culture solution containing 0.1% of ethanol was similarly cultured. The culture solutions were stained by the Trypan Blue staining method and the surviving cell number was measured to determine the degree of inhibition relative to the control. The results are given in the following Table.

Table: Inhibition of Proliferation Using Human Myelogenous Leukemic Cells

| Compound | Surviving Cell Number (cells/ml) | Degree of Inhibition |
|---|---|---|
| Control | $44.0 \times 10^4$ | - |
| (5Z,9Z,14EZ)-11-Oxoprosta-5,9,12,14-tetraenoic Acid (Compound of the Invention) | $3 \times 10^4$ | 93.2 |
| $PGD_2$ (Known Compound) | $19.5 \times 10^4$ | 55.6 |

With regard to the pharmacological effects typical of other prostaglandins, for example, in the intravenous administration to the allobarbital-anesthetized dog, (5Z,9Z,14EZ)-11-oxoprosta-5,9,12,14-tetraenoic acid did not cause blood pressure depression at a dosage of 100 μg/kg animal body weight, whereas $PGD_2$ caused blood pressure depression by 26 mm Hg at a dosage of 10 μg/kg animal body weight and its effect lasted for 6 minutes.

The acute toxicity of the compounds of the present invention was >50 mg/kg by intraperitoneal administration, so that the prostaglandin D derivatives of the present invention may be considered to be sufficiently safe and suitable for medical use. For example, the $LD_{50}$ value for (5Z,9Z,14EZ)-11-oxoprosta-5,9,12,14-tetraenoic acid was 78 mg/kg by intraperitoneal administration in mice.

Preferred compounds of the general formula (I) of the present invention are, for example, as follows:

17-methyl-9-deoxy-$\Delta^9$-$PGD_2$,

17-ethyl-9-deoxy-$\Delta^9$-$PGD_2$,

17,20-dimethyl-9-deoxy-$\Delta^9$-$PGD_2$,

17-ethyl-20-methyl-9-deoxy-$\Delta^9$-$PGD_2$,

17-methyl-20-ethyl-9-deoxy-$\Delta^9$-$PGD_2$,

17,20-diethyl-9-deoxy-$\Delta^9$-$PGD_2$,

15-cyclobutyl-16,17,18,19,20-pentanor-9-deoxy-$\Delta^9$-$PGD_2$,

15-(1-propylcyclobutyl)-16,17,18,19,20-pentanor-9-deoxy-$\Delta^9$-$PGD_2$,

15-(1-butylcyclobutyl)-16,17,18,19,20-pentanor-9-deoxy-$\Delta^9$-$PGD_2$,

15-(3-ethylcyclobutyl)-16,17,18,19,20-pentanor-9-deoxy-$\Delta^9$-$PGD_2$,

15-(3-propylcyclobutyl)-16,17,18,19,20-pentanor-9-deoxy-$\Delta^9$-$PGD_2$,

15-cyclopentyl-16,17,18,19,20-pentanor-9-deoxy-$\Delta^9$-$PGD_2$,

16-cyclopentyl-17,18,19,20-tetranor-9-deoxy-$\Delta^9$-$PGD_2$,

17-cyclopentyl-18,19,20-trinor-9-deoxy-$\Delta^9$-$PGD_2$,

15-(3-ethylcyclopentyl)-16,17,18,19,20-pentanor-9-deoxy-$\Delta^9$-$PGD_2$,

15-(3-propylcyclopentyl)-16,17,18,19,20-pentanor-9-deoxy-$\Delta^9$-$PGD_2$,

15-(3-butylcyclopentyl)-16,17,18,19,20-pentanor-9-deoxy-$\Delta^9$-$PGD_2$,

15-cyclohexyl-16,17,18,19,20-pentanor-9-deoxy-$\Delta^9$-$PGD_2$,

16-cyclohexyl-17,18,19,20-tetranor-9-deoxy-$\Delta^9$-$PGD_2$,

17-cyclohexyl-18,19,20-trinor-9-deoxy-$\Delta^9$-$PGD_2$,

15-(4-methylcyclohexyl)-16,17,18,19,20-pentanor-9-deoxy-$\Delta^9$-$PGD_2$,

15-(4-ethylcyclohexyl)-16,17,18,19,20-pentanor-9-deoxy-$\Delta^9$-$PGD_2$,

15-(4-propylcyclohexyl)-16,17,18,19,20-pentanor-9-deoxy-$\Delta^9$-$PGD_2$,

15-(4-butylcyclohexyl)-16,17,18,19,20-pentanor-9-deoxy-$\Delta^9$-$PGD_2$,

and the corresponding methyl and ethyl esters, and the corresponding 9-deoxy-$\Delta^9$-$PGD_1$ analogues i.e. compounds of the general formula (I) wherein X represents an ethylene group .

Preferred compounds of the general formula (II) of the present invention are, for example, as follows:

(5Z,9Z,14EZ)-11-oxo-20-norprosta-5,9,12,14-tetraenoic acid,

(5Z,9Z,14EZ)-11-oxo-prosta-5,9,12,14-tetraenoic acid,

(5Z,9Z,14EZ)-11-oxo-16-methylprosta-5,9,12,14-tetraenoic acid,

(5Z,9Z,14EZ)-11-oxo-17-methylprosta-5,9,12,14-tetraenoic acid,

(5Z,9Z,14EZ)-11-oxo-18-methylprosta-5,9,12,14-tetraenoic acid,

(5Z,9Z,14EZ)-11-oxo-16,16-dimethylprosta-5,9,12,14-tetraenoic acid,

(5Z,9Z,14EZ)-11-oxo-16-ethylprosta-5,9,12,14-tetraenoic acid,

(5Z,9Z,14EZ)-11-oxo-17-ethylprosta-5,9,12,14-tetraenoic acid,

(5Z,9Z,14EZ)-11-oxo-20-methylprosta-5,9,12,14-tetraenoic acid,

(5Z,9Z,14EZ)-11-oxo-16,20-dimethylprosta-5,9,12,14-tetraenoic acid,

(5Z,9Z,14EZ)-11-oxo-17,20-dimethylprosta-5,9,12,14-tetraenoic acid,

(5Z,9Z,14EZ)-11-oxo-16-ethyl-20-methylprosta-5,9,12,14-tetraenoic acid,

(5Z,9Z,14EZ)-11-oxo-17-ethyl-20-methylprosta-5,9,12,14-tetraenoic acid,

(5Z,9Z,14EZ)-11-oxo-20-ethylprosta-5,9,12,14-tetraenoic acid,

(5Z,9Z,14EZ)-11-oxo-17-methyl-20-ethylprosta-5,9,12,14-tetraenoic acid,

(5Z,9Z,14EZ)-11-oxo-17,20-diethylprosta-5,9,12,14-tetraenoic acid,

(5Z,9Z,14EZ)-11-oxo-15-cyclobutyl-16,17,18,19,20-pentanorprosta-5,9,12,14-tetraenoic acid,

(5Z,9Z,14EZ)-11-oxo-15-(1-propylcyclobutyl)-16,17,18,19,20-pentanorprosta-5,9,12,14-tetraenoic acid,

(5Z,9Z,14EZ)-11-oxo-15-(1-butylcyclobutyl)-16,17,18,19,20-pentanorprosta-5,9,12,14-tetraenoic acid,

(5Z,9Z,14EZ)-11-oxo-15-(3-ethylcyclobutyl)-16,17,18,19,20-pentanorprosta-5,9,12,14-tetraenoic acid, (5Z,9Z,14EZ)-11-oxo-15-(3-propylcyclobutyl)-16,17,18,19,20-pentanorprosta-5,9,12,14-tetraenoic acid, (5Z,9Z,14EZ)-11-oxo-15-cyclopentyl-16,17,18,19,20-pentanorprosta-5,9,12,14-tetraenoic acid, (5Z,9Z,14EZ)-11-oxo-16-cyclopentyl-17,18,19,20-tetranorprosta-5,9,12,14-tetraenoic acid, (5Z,9Z,14EZ)-11-oxo-17-cyclopentyl-18,19,20-trinorprosta-5,9,12,14-tetraenoic acid, (5Z,9Z,14EZ)-11-oxo-15-(3-ethylcyclopentyl)-16,17,18,19,20-pentanorprosta-5,9,12,14-tetraenoic acid, (5Z,9Z,14EZ)-11-oxo-15-(3-propylcyclopentyl)-16,17,18,19,20-pentanorprosta-5,9,12,14-tetraenoic acid, (5Z,9Z,14EZ)-11-oxo-15-(3-butylcyclopentyl)-16,17,18,19,20-pentanorprosta-5,9,12,14-tetraenoic acid, (5Z,9Z,14EZ)-11-oxo-15-cyclohexyl-16,17,18,19,20-pentanorprosta-5,9,12,14-tetraenoic acid, (5Z,9Z,14EZ)-11-oxo-16-cyclohexyl-17,18,19,20-tetranorprosta-5,9,12,14-tetraenoic acid, (5Z,9Z,14EZ)-11-oxo-17-cyclohexyl-18,19,20-trinorprosta-5,9,12,14-tetraenoic acid, (5Z,9Z,14EZ)-11-oxo-15-(4-methylcyclohexyl)-16,17,18,19,20-pentanorprosta-5,9,12,14-tetraenoic acid, (5Z,9Z,14EZ)-11-oxo-15-(4-ethylcyclohexyl)-16,17,18,19,20-pentanorprosta-5,9,12,14-tetraenoic acid, (5Z,9Z,14EZ)-11-oxo-15-(4-propylcyclohexyl)-16,17,18,19,20-pentanorprosta-5,9,12,14-tetraenoic acid,

(5Z,9Z,14EZ)-11-oxo-15-(4-butylcyclohexyl)-16,17,18,19,20-pentanorprosta-5,9,12,14-tetraenoic acid,

(5Z,9Z,14EZ)-11-oxo-16-phenyl-17,18,19,20-tetranorprosta-5,9,12,14-tetraenoic acid,

(5Z,9Z,14EZ)-11-oxo-17-phenyl-18,19,20-trinorprosta-5,9,12,14-tetraenoic acid,

(5Z,9Z,14EZ)-11-oxo-16-(4-methylphenyl)-17,18,19,20-tetranorprosta-5,9,12,14-tetraenoic acid,

(5Z,9Z,14EZ)-11-oxo-16-(4-ethylphenyl)-17,18,19,20-tetranorprosta-5,9,12,14-tetraenoic acid,

(5Z,9Z,14EZ)-11-oxo-16-phenoxy-17,18,19,20-tetranorprosta-5,9,12,14-tetraenoic acid,

(5Z,9Z,14EZ)-11-oxo-17-phenoxy-18,19,20-trinorprosta-5,9,12,14-tetraenoic acid,

(5Z,9Z,14EZ)-11-oxo-16-(3-chlorophenoxy)-17,18,19,20-tetranorprosta-5,9,12,14-tetraenoic acid,

(5Z,9Z,14EZ)-11-oxo-16-(4-chlorophenoxy)-17,18,19,20-tetranorprosta-5,9,12,14-tetraenoic acid,

(5Z,9Z,14EZ)-11-oxo-16-(3-trifluoromethylphenoxy)-17,18,19,20-tetranorprosta-5,9,12,14-tetraenoic acid,

(5Z,9Z,14EZ)-11-oxo-16-(4-trifluoromethylphenoxy)-17,18,19,20-tetranorprosta-5,9,12,14-tetraenoic acid,

(5Z,9Z,14EZ)-11-oxo-16-(4-methylphenoxy)-17,18,19,20-tetranorprosta-5,9,12,14-tetraenoic acid,

(5Z,9Z,14EZ)-11-oxo-16-(4-ethylphenoxy)-17,18,19,20-tetranorprosta-5,9,12,14-tetraenoic acid, and the corresponding methyl and ethyl esters, and the corresponding compounds wherein X represents an ethylene group.

The following Reference Examples and Examples illustrate the preparation of compounds of the present invention. In the Reference Examples and Examples, 'TLC', 'NMR', 'IR' and 'Mass' represent 'Thin layer chromatography','Nuclear magnetic resonance spectrum', 'Infrared absorption spectrum' and 'Mass spectrum', respectively. The solvents in parentheses specified in chromatographic separations show the developing solvents used : ratios are by volume. Except when specified otherwise, infrared absorption spectra were recorded by the liquid film method and nuclear magnetic resonance spectra were recorded in deuterochloroform ($CDCl_3$) solution.

Reference Example 1

(5Z,13E)-(9α,11α,15S,17S)-9,11-dihydroxy-15-(tetrahydropyran-2-yloxy)-17,20-dimethylprosta-5,13-dienoic acid methyl ester.

To a mixture of 1.1 g of 17S,20-dimethyl-$PGF_{2\alpha}$ methyl ester in 25 ml of dry methylene chloride, and 409 mg of phenylboric acid, was added a molecular sieve 4A (20 - 25 grains) and the mixture obtained was refluxed for one hour. After cooling to room temperature, the reaction mixture was filtered and to the filtrate obtained were added 0.382 ml of 2,3-dihydropyran and 70.3 mg of pyridine p-toluenesulfonate and then the mixture was stirred overnight. To the reaction mixture was added 90 ml of ethyl acetate and the mixture was washed successively with a saturated aqueous solution of sodium bicarbonate and a saturated aqueous solution of sodium chloride,

dried over anhydrous magnesium sulfate and concentrated under reduced pressure. The residue obtained was dissolved in 10 ml of methanol and thereto were added 40 ml of water and 4.0 ml of a 30% aqueous solution of hydrogen peroxide and the solution was warmed to 45 - 48°C. After cooling to room temperature, the reaction mixture was concentrated under reduced pressure; to the residue obtained was added 50 ml of ethyl acetate and then the solution obtained was washed successively with water and a saturated aqueous solution of sodium chloride, dried over anhydrous magnesium sulfate and concentrated under reduced pressure. The residue was purified by column chromatography on silica gel (eluent : ethyl acetate:cyclohexane=1:1) to give 920 mg of the title compound having the following physical characteristics:

TLC (benzene:ethyl acetate=1:2): Rf=0.43;

NMR: δ=5.7-5.2 (4H, m), 4.7 (1H, m), 3.6 (3H, s), 4.2-3.3 (5H, m), 1.0-0.8 (6H, m);

IR: ν=3440, 2938, 1737 $cm^{-1}$;

Mass: m/e=462.

By the same procedure as described in Reference Example 1, the following compounds were prepared.

(a) (5Z,13E)-(9α,11α,15S)-9,11-dihydroxy-15-(tetrahydropyran-2-yloxy)-15-(3-propylcyclopentyl)-16,17,18,19,20-pentanorprosta-5,13-dienoic acid methyl ester

Starting material: 15-(3-propylcyclopentyl)-16,17,18,19,20-

pentanor-$PGF_{2\alpha}$ methyl ester;

TLC (benzene:ethyl acetate=1:2): Rf=0.45;

NMR: δ=5.7-5.2 (4H, m), 4.7 (1H, m), 3.7 (3H, s), 4.2-3.3 (5H, m), 0.9 (3H, t);

IR: ν=3440, 2935, 1735 $cm^{-1}$;

Mass: m/e=474.

(b) (5Z,13E)-(9α,11α,15S)-9,11-dihydroxy-15-(tetrahydropyran-2-yloxy)-16-phenyl-17,18,19,20-tetranorprosta-5,13-dienoic acid methyl ester

Starting material: 16-phenyl-17,18,19,20-tetranor-$PGF_{2\alpha}$ methyl ester;

TLC (benzene:ethyl acetate=1:2): Rf=0.40;

NMR: δ=7.5-7.1 (5H, m), 5.7-5.2 (4H, m), 4.7 (1H, m), 3.6 (3H, s), 4.2-3.3 (5H, m);

IR: ν=3350, 2937, 1737 $cm^{-1}$;

Mass: m/e=454.

(c) (5Z,13E)-(9α,11α,15R)-9,11-dihydroxy-15-(tetrahydropyran-2-yloxy)-16-(3-chlorophenoxy)-17,18,19,20-tetranorprosta-5,13-dienoic acid methyl ester

Starting material: 16-(3-chlorophenoxy)-17,18,19,20-tetranor-$PGF_{2\alpha}$ methyl ester;

TLC (benzene:ethyl acetate=1:2): Rf=0.40;

NMR: δ=7.3-6.7 (4H, m), 5.7-5.2 (4H, m), 4.7-3.8 (8H, m), 3.7 (3H, s);

IR: ν=3440, 2935, 1737, 1600 $cm^{-1}$;

Mass: m/e=504.

(d) (5Z,13E)-(9α,11α,15R)-9,11-dihydroxy-15-(tetrahydropyran-2-yloxy)-16,16-dimethylprosta-5,13-dienoic acid methyl ester

Starting material: 16,16-dimethyl-$PGF_{2\alpha}$ methyl ester;

TLC (ethyl acetate): Rf=0.65;

NMR: δ=5.7-5.1 (4H, m), 4.6 (1H, m), 3.6 (3H, s), 1.0-0.8 (9H, m);

IR: ν=3440, 2940, 1740, 1440 $cm^{-1}$.

Reference Example 2

(5Z,13E)-(9α,11α,15S,17S)-9-hydroxy-11-benzoyloxy-15-(tetrahydropyran-2-yloxy)-17,20-dimethylprosta-5,13-dienoic acid methyl ester.

To a solution of 970 mg of the 9,11-dihydroxy compound (prepared in Reference Example 1) in 40 ml of methylene chloride, was added 1.63 ml of pyridine and the solution was cooled to -30 to -40°C. To the solution was added dropwise slowly 0.71 ml of benzoyl chloride in 3 ml of methylene chloride and the solution was stirred for 20 minutes at the same temperature. After adding 12.1 ml of methanol, the solution was further stirred for 20 minutes and warmed to 0°C. To the reaction mixture was added 20 ml of methylene chloride and the mixture was washed successively with water and a saturated aqueous solution of sodium chloride,

dried over anhydrous magnesium sulfate and concentrated under reduced pressure. The residue was purified by column chromatography on silica gel (eluent: ethyl acetate:cyclohexane=3:1) to give 950 mg of the title compound having the following physical characteristics:

TLC (cyclohexane:ethyl acetate=2:1): Rf=0.48;

NMR: δ=8.1-7.8 (2H, m), 7.6-7.2 (3H, m), 5.8-5.2 (4H, m), 5.2-4.9 (1H, m), 4.5 (1H, m), 3.6 (3H, s), 1.0-0.8 (6H, m);

IR: ν=3530, 2940, 1737, 1720, 1275 $cm^{-1}$.

By the same procedure as described in Reference Example 2, the following compounds were prepared.

(a) (5Z,13E)-(9α,11α,15S)-9-hydroxy-11-benzoyloxy-15-(tetrahydropyran-2-yloxy)-15-(3-propylcyclopentyl)-16,17,18,19,20-pentanorprosta-5,13-dienoic acid methyl ester

Starting material: the 9,11-dihydroxy compound prepared in Reference Example 1(a);

TLC (cyclohexane:ethyl acetate=2:1): Rf=0.50;

NMR: δ=8.1-7.8 (2H, m), 7.6-7.2 (3H, m), 5.8-5.2 (4H, m), 5.2-4.9 (1H, m), 4.6 (1H, m), 3.6 (3H, s), 0.9 (3H, t);

IR: ν=3530, 2935, 1735, 1720 $cm^{-1}$.

(b) (5Z,13E)-(9α,11α,15S)-9-hydroxy-11-benzoyloxy-15-(tetrahydropyran-2-yloxy)-16-phenyl-17,18,19,20-tetranorprosta-5,13-dienoic acid methyl ester

Starting material: the 9,11-dihydroxy compound prepared in Reference Example 1(b);

TLC (cyclohexane:ethyl acetate=2:1): Rf=0.45;

NMR: δ=8.1-7.1 (10H, m), 5.8-5.2 (4H, m), 5.2-4.9 (1H, m), 4.6 (1H, m), 3.6 (3H, s);

IR: ν=3540, 2935, 1737, 1720 $cm^{-1}$.

(c) (5Z,13E)-(9α,11α,15R)-9-hydroxy-11-benzoyloxy-15-(tetrahydropyran-2-yloxy)-16-(3-chlorophenoxy)-17,18,19,20-tetranorprosta-5,13-dienoic acid methyl ester

Starting material: the 9,11-dihydroxy compound prepared in Reference Example 1(c);

TLC (cyclohexane:ethyl acetate=2:1): Rf=0.45;

NMR: δ=8.1-7.8 (2H, m), 7.6-6.7 (7H, m), 5.8-5.2 (4H, m), 5.2-4.9 (1H, m), 3.7 (3H, s);

IR: ν=3530, 2935, 1736, 1720, 1600, 1273 $cm^{-1}$.

(d) (5Z,13E)-(9α,11α,15R)-9-hydroxy-11-benzoyloxy-15-(tetrahydropyran-2-yloxy)-16,16-dimethylprosta-5,13-dienoic acid methyl ester

Starting material: the 9,11-dihydroxy compound prepared in Reference Example 1(d);

TLC (cyclohexane:ethyl acetate=2:1): Rf=0.49;

NMR: δ=8.1-7.8 (2H, m), 7.6-7.2 (3H, m), 5.8-5.2 (4H, m), 5.2-4.9 (1H, m), 4.5 (1H, m), 3.6 (3H, s), 1.0-0.8 (9H, m).

0097023

Reference Example 3

(5Z,13E)-(9α,11α,15S,17S)-9,15-bis(tetrahydropyran-2-yloxy)-11-benzoyloxy-17,20-dimethylprosta-5,13-dienoic acid methyl ester.

To a solution of 4.5 g of the 9-hydroxy compound (prepared in Reference Example 2) in 20 ml of methylene chloride, was added dropwise one ml of 2,3-dihydropyran and then was added 10 mg of p-toluenesulfonic acid. After stirring for 15 minutes, 0.1 ml of triethylamine was added to the reaction mixture and the mixture was concentrated under reduced pressure. The residue was purified by column chromatography on silica gel (eluent: n-hexane:ethyl acetate=5:1) to give 4.0 g of the title compound having the following physical characteristics:

TLC (n-hexane:ethyl acetate=2:1): Rf=0.47;

NMR: δ=8.1-7.9 (2H, m), 7.6-7.2 (3H, m), 5.7-5.2 (4H, m), 5.2-4.9 (1H, m), 4.75-4.4 (2H, m), 3.7 (3H, s), 1.0-0.7 (6H, m);

IR: ν=2940, 1736, 1720 $cm^{-1}$.

By the same procedure as described in Reference Example 3, the following compounds were prepared.

(a) (5Z,13E)-(9α,11α,15S)-9,15-bis(tetrahydropyran-2-yloxy)-11-benzoyloxy-15-(3-propylcyclopentyl)-16,17,18,19,20-pentanorprosta-5,13-dienoic acid methyl ester

Starting material: the 9-hydroxy compound prepared in Reference Example 2(a);

TLC (n-hexane:ethyl acetate=2:1): Rf=0.49;

NMR: δ=8.1-7.9 (2H, m), 7.6-7.2 (3H, m), 5.7-5.2 (4H, m), 5.3-4.9 (1H, m), 4.8-4.4 (2H, m), 3.7 (3H, s), 0.89 (3H, t);

IR: ν=2940, 1735, 1722 $cm^{-1}$.

(b) (5Z,13E)-(9α,11α,15S)-9,15-bis(tetrahydropyran-2-yloxy)-11-benzoyloxy-16-phenyl-17,18,19,20-tetranorprosta-5,13-dienoic acid methyl ester

Starting material: the 9-hydroxy compound prepared in Reference Example 2(b);

TLC (n-hexane:ethyl acetate=2:1): Rf=0.45;

NMR: δ=8.1-7.1 (10H, m), 5.7-5.2 (4H, m), 5.2-4.9 (1H, m), 4.7-4.5 (2H, m), 3.7 (3H, s);

IR: ν=2935, 1736, 1720 $cm^{-1}$.

(c) (5Z,13E)-(9α,11α,15R)-9,15-bis(tetrahydropyran-2-yloxy)-11-benzoyloxy-16-(3-chlorophenoxy)-17,18,19,20-tetranorprosta-5,13-dienoic acid methyl ester

Starting material: the 9-hydroxy compound prepared in Reference Example 2(c);

TLC (n-hexane:ethyl acetate=2:1): Rf=0.45;

NMR: δ=8.1-7.9 (2H, m), 7.6-6.7 (7H, m), 5.7-5.2 (4H, m), 5.2-4.9 (1H, m), 3.7 (3H, s);

IR: ν=2940, 1737, 1720, 1600 $cm^{-1}$.

(d) (5Z,13E)-(9α,11α,15R)-9,15-bis(tetrahydropyran-2-yloxy)-11-benzoyloxy-16,16-dimethylprosta-5,13-dienoic acid methyl ester

Starting material: the 9-hydroxy compound prepared in Reference Example 2(d);

TLC (cyclohexane:ethyl acetate=2:1) : Rf=0.57;

NMR: δ=8.1-7.8 (2H, m), 7.6-7.2 (3H, m), 5.8-5.2 (4H, m), 5.2-4.9 (1H, m), 4.7-4.4 (2H, m), 3.6 (3H, s), 1.0-0.8 (9H, m).

Reference Example 4

(5Z,13E)-(9α,11α,15S,17S)-9,15-bis(tetrahydropyran-2-yloxy)-11-hydroxy-17,20-dimethylprosta-5,13-dienoic acid.

To a solution of 1.26 g of the 11-benzoyloxy compound (prepared in Reference Example 3) in 10 ml of methanol, was added 3.8 ml of a 2N aqueous solution of potassium hydroxide, and the solution obtained was stirred for 3 hours at 50 - 60°C. After cooling to room temperature, the reaction mixture was concentrated under reduced pressure until its volume was reduced to half, and then 50 ml of a 20% aqueous solution of ammonium chloride was added thereto. The solution was extracted with 50 ml of ethyl acetate twice (total: 100 ml) and the extract was washed with a saturated aqueous solution of sodium chloride, dried over anhydrous magnesium sulfate and concentrated under reduced pressure to

give 1.04 g of the title compound as crude product having the following physical characteristics. The product obtained was used in the procedure of Reference Example 5 without purification.

TLC (benzene:ethyl acetate=2:1): Rf=0.07.

By the same procedure as described in Reference Example 4, the following compounds were prepared.

(a) (5Z,13E)-(9α,11α,15S)-9,15-bis(tetrahydropyran-2-yloxy)-11-hydroxy-15-(3-propylcyclopentyl)-16,17,18,19,20-pentanorprosta-5,13-dienoic acid

Starting material: the 11-benzoyloxy compound prepared in Reference Example 3(a);

TLC (benzene:ethyl acetate=2:1): Rf=0.07.

(b) (5Z,13E)-(9α,11α,15S)-9,15-bis(tetrahydropyran-2-yloxy)-11-hydroxy-16-phenyl-17,18,19,20-tetranorprosta-5,13-dienoic acid

Starting material: the 11-benzoyloxy compound prepared in Reference Example 3(b);

TLC (benzene:ethyl acetate=2:1): Rf=0.08.

(c) (5Z,13E)-(9α,11α,15R)-9,15-bis(tetrahydropyran-2-yloxy)-11-hydroxy-16-(3-chlorophenoxy)-17,18,19,20-tetranorprosta-5,13-dienoic acid

Starting material: the 11-benzoyloxy compound prepared in Reference Example 3(c);

TLC (benzene:ethyl acetate=2:1): Rf=0.06.

(d) (5Z,13E)-(9α,11α,15R)-9,15-bis(tetrahydropyran-2-yloxy)-11-hydroxy-16,16-dimethylprosta-5,13-dienoic

acid

Starting material: the 11-benzoyloxy compound prepared in Reference Example 3(d);

TLC (cyclohexane:ethyl acetate=2:1): Rf=0.08;

NMR: δ=5.7-5.3 (4H, m), 4.7-4.5 (2H, m), 1.0-0.8 (9H, m);

Mass: m/e=448, 431, 367, 346.

Reference Example 5

(5Z,13E)-(9α,15S,17S)-11-oxo-9,15-bis(tetrahydropyran-2-yloxy)-17,20-dimethylprosta-5,13-dienoic acid.

A solution of 1.04 g of the 11-hydroxy compound (prepared in Reference Example 4) in 10 ml of acetone was cooled to -30°C and Jones reagent (prepared from 378 mg of chromium trioxide, 0.331 ml of concentrated sulfuric acid and 1.41 ml of water) was added thereto dropwise, and then the solution was stirred for 30 minutes at the same temperature. After adding one ml of isopropyl alcohol and 100 ml of water, successively, the reaction mixture was extracted with 50 ml of diethyl ether three times (total: 150 ml) and the extract was washed successively with water and a saturated aqueous solution of sodium chloride, dried over anhydrous magnesium sulfate and concentrated under reduced pressure. The residue was purified by column chromatography on silica gel (eluent: cyclohexane:ethyl acetate=2:1) to give

652 mg of the title compound having the following physical characteristics:

TLC (benzene:ethyl acetate=1:2): Rf=0.71;

NMR: δ=10.1 (1H, s), 5.7-5.2 (4H, m), 4.8-4.5 (2H, m), 1.0-0.7 (6H, m);

Mass: m/e=464, 446.

By the same procedure as described in Reference Example 5, the following compounds were prepared.

(a) (5Z,13E)-(9α,15S)-11-oxo-9,15-bis(tetrahydropyran-2-yloxy)-15-(3-propylcyclopentyl)-16,17,18,19,20-pentanorprosta-5,13-dienoic acid

Starting material: the 11-hydroxy compound prepared in Reference Example 4(a);

TLC (benzene:ethyl acetate=1:2): Rf=0.71;

NMR: δ=10.3 (1H, s), 5.7-5.2 (4H, m), 4.8-4.5 (2H, m), 0.88 (3H, t);

Mass: m/e=476, 458.

(b) (5Z,13E)-(9α,15S)-11-oxo-9,15-bis(tetrahydropyran-2-yloxy)-16-phenyl-17,18,19,20-tetranorprosta-5,13-dienoic acid

Starting material: the 11-hydroxy compound prepared in Reference Example 4(b);

TLC (benzene:ethyl acetate=1:2): Rf=0.70;

NMR: δ=10.3 (1H, s), 7.5-7.1 (5H, m), 5.7-5.2 (4H, m), 4.8-4.5 (2H, m);

Mass: m/e=456, 438.

(c) (5Z,13E)-(9α,15R)-11-oxo-9,15-bis(tetrahydropyran-2-yloxy)-16-(3-chlorophenoxy)-17,18,19,20-tetranorprosta-5,13-dienoic acid

Starting material: the 11-hydroxy compound prepared in Reference Example 4(c);

TLC (benzene:ethyl acetate=1:2): Rf=0.70;

NMR: δ=10.3 (1H, s), 7.3-6.7 (4H, m), 5.7-5.2 (4H, m);

Mass: m/e=506, 488.

(d) (5Z,13E)-(9α,15R)-11-oxo-9,15-bis(tetrahydropyran-2-yloxy)-16,16-dimethylprosta-5,13-dienoic acid

Starting material: the 11-hydroxy compound prepared in Reference Example 4(d);

TLC (cyclohexane:ethyl acetate=1:1): Rf=0.44;

NMR: δ=5.78-5.30 (4H, m), 4.85-3.35 (8H, m), 1.00-0.80 (9H, m);

IR: ν=2930, 1735, 1700 $cm^{-1}$;

Mass: m/e=447, 365, 345.

Reference Example 6

(5Z,13E)-(9α,15S,17S)-11-oxo-9,15-dihydroxy-17,20-dimethylprosta-5,13-dienoic acid (i.e. 17S,20-dimethyl-$PGD_2$)

A mixture of 600 mg of the 11,15-bis(tetrahydropyran-2-yloxy) compound (prepared in Reference Example 5), 1.15 ml of tetrahydrofuran and 10 ml of a 65% (v/v) aqueous solution of acetic acid was stirred for 8 minutes at

80°C. After adding 50 ml of ice-water, the reaction mixture was extracted with 50 ml of ethyl acetate three times (total: 150 ml) and the extract was washed successively with water and a saturated aqueous solution of sodium chloride, dried over anhydrous magnesium sulfate and concentrated under reduced pressure. Then, acetic acid was removed as the toluene azeotrope. The residue was purified by column chromatography on silica gel (eluent: cyclohexane:ethyl acetate=1:1 → ethyl acetate only) to give 262 mg of the title compound having the following physical characteristics:

TLC (chloroform:tetrahydrofuran:acetic acid=20:4:1): Rf=0.38;

NMR: δ=5.74-5.36 (4H, m), 4.50 (1H, m), 4.12 (1H, m), 2.82 (1H, m), 1.02-0.81 (6H, m);

IR: ν=3420, 2920, 1740, 1710 $cm^{-1}$;

Mass: m/e=362, 344.

By the same procedure as described in Reference Example 6, the following compounds were prepared.

(a) (5Z,13E)-(9α,15S)-11-oxo-9,15-dihydroxy-15-(3-propylcyclopentyl)-16,17,18,19,20-pentanorprosta-5,13-dienoic acid (i.e. 15-(3-propylcyclopentyl)-16,17,18,19,20-pentanor-$PGD_2$)

Starting material: the 9,15-bis(tetrahydropyran-2-yloxy) compound prepared in Reference Example 5(a);

TLC (chloroform:tetrahydrofuran:acetic acid=20:4:1): Rf=0.40;

NMR: δ=5.75-5.35 (4H, m), 4.55 (1H, m), 4.12 (1H, m), 2.80

(1H, m), 0.89 (3H, t);

IR: ν=3430, 2920, 1740, 1712 $cm^{-1}$;

Mass: m/e=374, 356.

(b) (5Z,13E)-(9α,15S)-11-oxo-9,15-dihydroxy-16-phenyl-17,18,19,20-tetranorprosta-5,13-dienoic acid (i.e. 16-phenyl-17,18,19,20-tetranor-$PGD_2$)

Starting material: the 9,15-bis(tetrahydropyran-2-yloxy) compound prepared in Reference Example 5(b);

TLC (chloroform:tetrahydrofuran:acetic acid=20:4:1): Rf=0.38;

NMR: δ=7.53-7.14 (5H, m), 5.73-5.36 (4H, m), 4.55 (1H, m), 4.20 (1H, m), 2.85 (1H, m);

IR: ν=3420, 2920, 1742, 1711 $cm^{-1}$;

Mass: m/e=354, 336.

(c) (5Z,13E)-(9α,15R)-11-oxo-9,15-dihydroxy-16-(3-chlorophenoxy)-17,18,19,20-tetranorprosta-5,13-dienoic acid (i.e. 16-(3-chlorophenoxy)-17,18,19,20-tetranor-$PGD_2$)

Starting material: the 9,15-bis(tetrahydropyran-2-yloxy) compound prepared in Reference Example 5(c);

TLC (chloroform:tetrahydrofuran:acetic acid=20:4:1): Rf=0.36;

NMR: δ=7.28-6.70 (4H, m), 5.75-5.35 (4H, m), 4.63-4.35 (2H, m), 3.94 (2H, d), 2.85 (1H, m);

IR: ν=3430, 2925, 1740, 1710, 1600 $cm^{-1}$;

Mass: m/e=404, 386.

(d) (5Z,13E)-(9α,15R)-11-oxo-9,15-dihydroxy-16,16-dimethylprosta-5,13-dienoic acid (i.e. 16,16-dimethyl-$PGD_2$)

Starting material: the 9,15-bis(tetrahydropyran-2-yloxy) compound prepared in Reference Example 5(d);

TLC (chloroform:tetrahydrofuran:acetic acid=20:2:1): Rf=0.08;

NMR: δ=5.90-5.35 (4H, m), 4.60-4.45 (1H, m), 3.90 (1H, d), 2.85 (1H, dd), 1.00-0.90 (9H, m);

IR: ν=2955-2920, 1860, 1730-1695, 1450 $cm^{-1}$;

Mass: m/e=362, 344, 301, 268.

Reference Example 7

(5Z,13E)-(9α,15S)-11-oxo-9,15-dihydroxyprosta-5,13-dienoic acid ethyl ester (i.e. $PGD_2$ ethyl ester)

Under an atmosphere of nitrogen, to a solution of 50 mg of $PGD_2$ (prepared by the method as described in Example 1 [B] in the specification of the Japanese Patent Kokoku No. 54-32773) in 0.5 ml of ethanol was added 29 mg of dicyclohexylcarbodiimide at 0°C, followed by 17 mg of 4-dimethylaminopyridine in 2.5 ml of methylene chloride added slowly dropwise. After warming to room temperature, the reaction mixture was stirred for 2 hours. The reaction mixture was diluted with diethyl ether, washed with water, dried and concentrated under reduced pressure. The residue

was purified by column chromatography on silica gel (eluent : diethyl ether:n-hexane=1:1→diethyl ether only) to give 16 mg of the title compound having the following physical characteristics:

TLC (chloroform:tetrahydrofuran:acetic acid=10:2:1): Rf=0.49;

NMR: δ=5.72-5.38 (4H, m), 4.52-4.40 (1H, m), 4.13 (2H, q), 4.20-4.04 (1H, m), 2.94-2.88 (1H, m), 1.25 (3H, t), 1.0-0.8 (3H, m);

IR (chloroform solution): ν=3500, 2930, 1730, 1720, 1370, 965 $cm^{-1}$;

Mass: m/e=380 ($M^+$), 362, 344.

By the same procedure as described in Reference Example 7, the following compounds were prepared.

(a) (13E)-(9α,15S)-11-oxo-9,15-dihydroxyprost-13-enoic acid ethyl ester (i.e. $PGD_1$ ethyl ester)

Starting material: $PGD_1$ (prepared by the method as described in Example 2 [B] in the specification of the Japanese Patent Kokoku No. 54-32773);

TLC (chloroform:tetrahydrofuran:acetic acid=10:2:1): Rf=0.50;

NMR: δ=5.7-5.3 (2H, m), 4.5 (1H, m), 4.10 (2H, q), 4.21-4.02 (1H, m), 0.90 (3H, t);

IR: ν=3420, 2930, 1730, 1720 $cm^{-1}$;

Mass: m/e=364, 346.

(b) (5Z,13E)-(9α,15S,17S)-11-oxo-9,15-dihydroxy-17,20-dimethylprosta-5,13-dienoic acid ethyl ester (i.e. 17S,20-dimethyl-$PGD_2$ ethyl ester)

Starting material: 17S,20-dimethyl-$PGD_2$ prepared in Reference Example 6;

TLC (chloroform:tetrahydrofuran:acetic acid=10:2:1): Rf=0.50;

NMR: $\delta$=5.75-5.35 (4H, m), 4.53-4.40 (1H, m), 4.13 (2H, q), 4.20-4.03 (1H, m), 2.94-2.88 (1H, m), 1.0-0.8 (6H, m);

IR: $\nu$=3500, 2930, 1730, 1720 $cm^{-1}$;

Mass: m/e=408, 390, 372.

Example 1

(5Z,9Z,13E)-(15S,17S)-11-oxo-15-hydroxy-17,20-dimethylprosta-5,9,13-trienoic acid (i.e. 17S,20-dimethyl-9-deoxy-$\Delta^9$-$PGD_2$)

To a solution of 53 mg of the $PGD_2$ compound (prepared in Reference Example 6) in 0.7 ml of ethanol was added 70 ml of a tris hydrochloric acid buffer solution (prepared from 17.5 ml of a 0.2M aqueous solution of tris(hydroxymethyl)aminomethane, 31.5 ml of a 0.1N hydrochloric acid and 21 ml of water) (pH=7.2) and the mixture was stirred for 48 hours at 37°C. The reaction mixture was extracted with 30 ml of ethyl acetate three times (total: 90 ml) and the extract was washed with a saturated aqueous solution of sodium chloride, dried over anhydrous magnesium sulfate and concentrated under reduced pressure. The residue was purified by column chromatography on silica gel (eluent: n-hexane:ethyl acetate=1:1) to give 5 mg of the title compound having the following physical characteristics:

TLC (chloroform:tetrahydrofuran:acetic acid=30:6:1): Rf=0.47;

NMR: δ=7.63 (1H, dd), 6.18 (1H, dd), 5.72-5.35 (4H, m), 4.20-4.05 (1H, m), 1.00-0.80 (6H, m);

IR: ν=3370, 2920, 1705, 1580 $cm^{-1}$;

Mass: m/e=362, 344.

By the same procedure as described in Example 1, the following compounds were prepared.

(a) (5Z,9Z,13E)-(15S)-11-oxo-15-hydroxy-15-(3-propylcyclopentyl)-16,17,18,19,20-pentanorprosta-5,9,13-trienoic acid i.e. 15-(3-propylcyclopentyl)-16,17,18,19,20-pentanor-9-deoxy-$\Delta^9$-$PGD_2$

Starting material: the $PGD_2$ compound prepared in Reference Example 6(a);

TLC (chloroform:tetrahydrofuran:acetic acid=30:6:1): Rf=0.49;

NMR: δ=7.64 (1H, dd), 6.17 (1H, dd), 5.75-5.35 (4H, m), 4.20-4.03 (1H, m), 0.88 (3H, t);

IR: ν=3360, 2920, 1705, 1582 $cm^{-1}$;

Mass: m/e=374, 356.

(b) (5Z,9Z,13E)-(15S,17S)-11-oxo-15-hydroxy-17,20-dimethylprosta-5,9,13-trienoic acid ethyl ester i.e. 17S,20-dimethyl-9-deoxy-$\Delta^9$-$PGD_2$ ethyl ester

Starting material: 17S,20-dimethyl-$PGD_2$ ethyl ester prepared in Reference Example 7(b);

NMR: δ=7.63 (1H, dd), 6.17 (1H, m), 5.75-5.35 (4H, m), 4.30-3.95 (3H, m), 1.00-0.80 (6H, m);

IR: ν=3400, 2920, 1738, 1580 $cm^{-1}$;

Mass: m/e=390, 372.

Example 2

(5Z,9Z,14EZ)-(17S)-11-oxo-17,20-dimethylprosta-5,9,12,14-tetraenoic acid

A mixture of 11 mg of the $PGD_2$ compound (prepared in Reference Example 6), 0.28 ml of 1N hydrochloric acid and 0.28 ml of tetrahydrofuran was refluxed for 20 minutes. To the reaction mixture was added 50 ml of diethyl ether and the solution thus obtained was washed with a saturated aqueous solution of sodium chloride, dried over anhydrous magnesium sulfate and concentrated under reduced pressure. The residue was purified by column chromatography on silica gel (eluent: n-hexane:ethyl acetate=2:1) to give 3 mg of the title compound having the following physical characteristics:

TLC (chloroform:tetrahydrofuran:acetic acid=30:6:1): Rf=0.64;

NMR: δ=7.48 (1H, dd), 6.97 (1H, d), 6.47-6.16 (3H, m), 5.56-5.30 (2H, m), 3.64-3.51 (1H, m), 1.00-0.80 (6H, m);

IR: ν=2920, 1727, 1702, 1626 $cm^{-1}$;

Mass: m/e=344.

By the same procedure as described in Example 2, the following compounds were prepared.

(a) (5Z,9Z,14EZ)-11-oxo-15-(3-propylcyclopentyl)-16,17,18,19,20-pentanorprosta-5,9,12,14-tetraenoic acid.

Starting material: the $PGD_2$ compound prepared in Reference Example 6(a);

TLC (chloroform:tetrahydrofuran:acetic acid=30:6:1): Rf=0.64;

NMR: δ=7.49 (1H, dd), 6.97 (1H, d), 6.47-6.16 (3H, m), 5.56-5.32 (2H, m), 3.64-3.50 (1H, m), 0.89 (3H, t);

IR: ν=2920, 1726, 1703, 1625 $cm^{-1}$;

Mass: m/e=356.

(b) (5Z,9Z,14EZ)-11-oxo-16-phenyl-17,18,19,20-tetranorprosta-5,9,12,14-tetraenoic acid

Starting material: the $PGD_2$ compound prepared in Reference Example 6(b);

TLC (chloroform:tetrahydrofuran:acetic acid=30:6:1): Rf=0.62;

NMR: δ=7.55-7.11 (6H, m), 6.97 (1H, d), 6.51-6.16 (3H, m), 5.57-5.30 (2H, m);

IR: ν=2920, 1728, 1703, 1625 $cm^{-1}$;

Mass: m/e=336.

(c) (5Z,9Z,14EZ)-11-oxo-16-(3-chlorophenoxy)-17,18,19,20-tetranorprosta-5,9,12,14-tetraenoic acid

Starting material: the $PGD_2$ compound prepared in Reference Example 6(c);

TLC (chloroform:tetrahydrofuran:acetic acid=30:6:1): Rf=0.63;

NMR: δ=7.51-6.70 (6H, m), 6.48-6.20 (3H, m), 5.61-5.31 (2H, m);

IR: ν=2930, 1728, 1702, 1625, 1600 $cm^{-1}$;

Mass: m/e=388, 386.

(d) (5Z,9Z,14EZ)-11-oxoprosta-5,9,12,14-tetraenoic acid

Starting material: $PGD_2$;

TLC (chloroform:tetrahydrofuran:acetic acid=30:6:1): Rf=0.62;

NMR: δ=7.47 (1H, dd), 6.96 (1H, d), 6.46-6.15 (3H, m), 5.55-5.28 (2H, m), 3.65-3.52 (1H, m), 0.90 (3H, t);

IR: ν=3600-2400, 2920, 1725, 1700, 1625 $cm^{-1}$;

Mass: m/e=316.

(e) (9Z,14EZ)-11-oxoprosta-9,12,14-trienoic acid

Starting material: $PGD_1$;

TLC (chloroform:tetrahydrofuran:acetic acid=30:6:1): Rf=0.63;

NMR: δ=7.46 (1H, dd), 6.95 (1H, d), 6.46-6.14 (3H, m), 3.62-3.50 (1H, m), 0.90 (3H, t);

IR: ν=2920, 1724, 1700, 1624 $cm^{-1}$;

Mass: m/e=318.

(f) (5Z,9Z,14EZ)-11-oxoprosta-5,9,12,14-tetraenoic acid ethyl ester

Starting material: $PGD_2$ ethyl ester prepared in Reference Example 7;

NMR: δ=7.47 (1H, dd), 6.96 (1H, d), 6.46-6.15 (3H, m), 5.54-5.27 (2H, m), 4.10 (2H, q), 3.58 (1H, m), 0.90 (3H, t);

IR: ν=2920, 1738, 1725, 1626 $cm^{-1}$;

Mass: m/e=344.

(g) (9Z,14EZ)-11-oxoprosta-9,12,14-trienoic acid ethyl

ester

Starting material: $PGD_1$ ethyl ester prepared in Reference Example 7(a);

NMR: δ=7.48 (1H, dd), 6.95 (1H, d), 6.46-6.14 (3H, m), 4.12 (2H, q), 0.90 (3H, t);

IR: ν=2920, 1738, 1726, 1627 $cm^{-1}$;

Mass: m/e=346.

(h) (5Z,9Z,14EZ)-( 17S)-11-oxo-17,20-dimethylprosta-5,9,12,14-tetraenoic acid ethyl ester

Starting material: 17S,20-dimethyl-$PGD_2$ ethyl ester prepared in Reference Example 7(b);

NMR: δ=7.47 (1H, dd), 6.96 (1H, dd), 6.46-6.14 (3H, m), 5.54-5.27 (2H, m), 4.10 (2H, q), 3.58 (1H, m), 1.0-0.80 (6H, m);

IR: ν=2920, 1735, 1720, 1628 $cm^{-1}$;

Mass: m/e=372.

(i) (5Z,9Z,14EZ)-11-oxoprosta-5,9,12,14-tetraenoic acid methyl ester

Starting material: $PGD_2$ methyl ester (prepared by esterifying a solution of $PGD_2$ in diethyl ether by adding a 0.5 M solution of diazomethane in diethyl ether until the solution was coloured pale yellow and then concentrating under reduced pressure);

TLC (ethyl acetate:n-hexane=1:3): Rf=0.44;

NMR: δ=7.46 (1H, dd), 6.94 (1H, d), 6.40-6.14 (3H, m),

5.53-5.28 (2H, m), 3.65 (3H, s), 3.57 (1H, m), 0.89 (3H, t);

IR: ν=2940, 1738, 1695, 1632, 1580 $cm^{-1}$;

Mass: m/e=330 ($M^+$), 299, 259.

(j) (9Z,14EZ)-11-oxoprosta-9,12,14-trienoic acid methyl ester

Starting material: $PGD_1$ methyl ester (prepared by esterifying $PGD_1$ with diazomethane);

TLC (ethyl acetate:n-hexane=1:2): Rf=0.64;

NMR: δ=7.50 (1H, dd), 6.91 (1H, d), 6.33 (1H, dd), 6.30-6.18 (2H, m), 3.65 (3H, s), 3.60-3.48 (1H, m);

IR: ν=2925, 1735, 1690, 1630, 1575 $cm^{-1}$;

Mass: m/e=332 ($M^+$), 301, 261.

(k) (5Z,9Z,14EZ)-11-oxo-16,16-dimethylprosta-5,9,12,14-tetraenoic acid methyl ester

Starting material: 16,16-dimethyl $PGD_2$ methyl ester [prepared by esterifying 16,16-dimethyl-$PGD_2$ prepared in Reference Example 6(d), with diazomethane];

TLC (cyclohexane:ethyl acetate=1:1): Rf=0.58;

NMR: δ=7.65-7.40 (1H, m), 7.05-6.90 (1H, m), 6.35 (1H, dd), 6.30-6.10 (2H, m), 5.60-5.25 (2H, m), 3.65 (3H, s), 3.65-3.50 (1H, m), 1.05 (6H, s), 0.85 (3H, t);

IR: ν=2940, 2850, 1730, 1680, 1620, 1425 $cm^{-1}$;

Mass: m/e=358 ($M^+$), 314, 273.

The present invention includes within its scope pharmaceutical compositions which comprise at least one prostaglandin D derivative of general formula (I) or (II), cyclodextrin clathrate thereof or, when $R^1$ represents a hydrogen atom, non-toxic salt thereof together with a pharmaceutical carrier or coating.

In clinical practice, for the prevention or the therapy (including therapy to secure remission) against leukemia or solid cancer, the compounds of the present invention will normally be administered systemically or partially; usually by oral or parenteral (e.g. intravenous, subcutaneous or intramuscular) administration.

Solid compositions for oral administration include compressed tablets, pills, dispersible powders and granules. In such solid compositions, one or more of the active compound(s) is, or are, admixed with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, and disintegrating agents, such as cellulose calcium gluconate. The tablets or pills may, if desired, be made into enteric film-coated or gastric film-coated tablets or pills, such as sugar-coated, gelatin-coated, hydroxypropylcellulose-coated

or hydroxypropylmethylcellulose phthalate-coated tablets or pills; two or more layers may be used.

The compositions for oral administration also include capsules of absorbable material such as gelatin, containing one or more of the active substances with or without the addition of diluents or excipients.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise at least one compound of the present invention.

Preparations according to the invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, and Polysorbate 80 (registered Trade Mark). These compositions may also include adjuvants

such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic lihiments such as ointments; suppositories for rectal administration; and pessaries for vaginal administraion. Such compositions are prepared by known methods.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when required for administration by injection; for oral administration the preparations will normally contain at least 0.1% by weight of active substance.

The dose to be administered is determined depending upon, for example, age, body weight, symptoms, the desired therapeutic effects, the route of administration, and the duration of the treatment.

In the human adult, the doses per person are generally between 5 mg and 500 mg by oral administration, and between 500 μg and 50 mg by parenteral administration for the prevention or the therapy (including therapy to secure remission) against leukemia or solid cancer, and can be administered up to several times per day.

As mentioned above, the doses to be used depend on various conditions. Therefore, there may be cases in which doses greater than the ranges specified above, or lower than the ranges specified above, may be used.

The following Examples illustrate pharmaceutical compositions according to the invention.

Example 3

A solution of one gram of (5Z,9Z,14EZ)-11-oxoprosta-5,9,12.14-tetraenoic acid in 5 ml of ethanol was well mixed with 5 g of microcrystalline cellulose. After drying the mixture sufficiently, 100 mg of magnesium stearate, 20 mg of silicon dioxide, 10 mg of talc, 200 mg of cellulose calcium gluconate (CCG) and further microcrystalline cellulose to produce 10 g of mixture were added. After mixing well to ensure homogeneity the mixture was punched out in conventional manner to obtain 100 tablets each containing 10 mg of the active ingredient.

Example 4

A α-cyclodextrin and ß-cyclodextrin clathrate of (5Z,9Z,14EZ)-11-oxoprosta-5,9,12,14-tetraenoic acid [prepared by dissolving 2.4 g of α-cyclodextrin and one g of ß-cyclodextrin in 300 ml of water, adding thereto 100 mg of (5Z,9Z,14EZ)-11-oxoprosta-5,9,12,14-tetraenoic acid, stirring well and then concentrating under reduced pressure] was dissolved in 150 ml of distilled water for injection. The solution was sterilized by filtration in conventional manner, placed in 1.5 ml portions in 5 ml ampoules and freeze-dried to obtain 100 ampoules of solid composition for injection each containing 1 mg of the active ingredient.

CLAIMS

CONTRACTING STATES: BE, CH, DE, FR, GB, IT, LU, LI, NL, SE

1. A prostaglandin D derivative of the general formula:

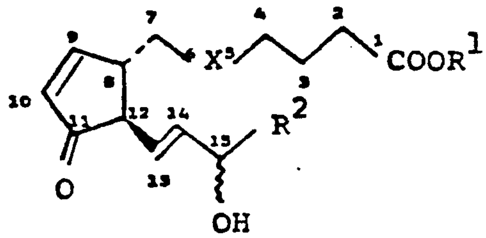

(I)

wherein $R^1$ represents a hydrogen atom or a straight-chain or branched-chain alkyl group of 1 - 12 carbon atoms, $R^2$ represents a group of the general formula:

(i) 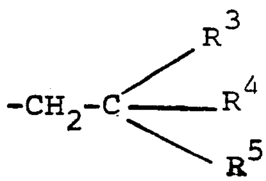 or

(ii) $-R^6-R^7$

wherein $R^3$ represents a straight-chain or branched-chain alkyl group of 1 - 4 carbon atoms, $R^4$ represents a hydrogen atom or a straight-chain or branched-chain alkyl group of 1 - 4 carbon atoms, $R^5$ represents a straight-chain or branched-chain alkyl group of 1 - 10 carbon atoms, $R^6$ represents a single bond or a straight-chain or branched-chain alkylene group of 1 - 5 carbon atoms, and $R^7$ represents a cycloalkyl group of 4 - 7 carbon atoms either unsubstituted or substituted by at least one straight-chain or branched-chain alkyl group of 1 - 8 carbon atoms,

X represents an ethylene group or a cis-vinylene group, the double bond between $C_9$ - $C_{10}$ is Z, and the double bond between $C_{13}$ - $C_{14}$ is E, or of the general formula:

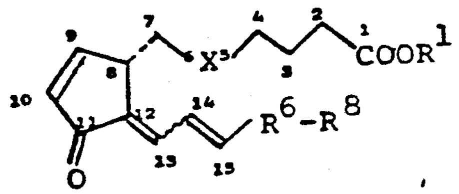

(II)

wherein $R^1$, $R^6$ and X are as hereinbefore defined, $R^8$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms or represents a phenyl group or phenoxy group either unsubstituted or substituted by at least one halogen atom, trifluoromethyl group or straight-chain or branched-chain alkyl group of 1 - 4 carbon atoms, the double bonds between $C_{12}$ - $C_{13}$ and between $C_{14}$ - $C_{15}$, which may be in the same or different configuration, are E, Z or a mixture thereof, and the double bond between $C_9$ - $C_{10}$ is Z, provided that when $R^6$ represents a single bond, $R^8$ does not represent a substituted or unsubstituted phenoxy group, and cyclodextrin clathrates of a compound of formula (I) or (II) and, when $R^1$ in the general formula (I) or (II) represents a hydrogen atom, non-toxic salts thereof.

2. A prostaglandin derivative according to claim 1, wherein $R^1$ represents a hydrogen atom or a straight-chain or branched-chain alkyl group of 1 - 4 carbon atoms.

3. A compound according to claim 1 or 2, wherein the hydroxy group attached to the 15-position carbon atom is in α-configuration.

4. A prostaglandin derivative according to claim 1, 2 or 3, wherein $R^2$ represents a 2-methylpentyl, 2-ethylpentyl, 2-methylhexyl, 2-ethylhexyl, 2-methylheptyl or 2-ethylheptyl group; wherein $R^2$ represents a cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl or 4-butylcyclohexyl group; wherein $R^6$-$R^8$ represents a butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl or 2-ethylheptyl group; wherein $R^6$-$R^8$ represents a cyclobutyl, 1-propylcyclobutyl, 1-butylcyclobutyl, 3-ethylcyclobutyl,

3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl or 4-butylcyclohexyl group; or wherein $R^6$-$R^8$ represents a benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl or 4-ethylphenoxymethyl group.

5. A prostaglandin derivative according to claim 4 which is (5Z,9Z,13E)-(15S,17S)-11-oxo-15-hydroxy-17,20-dimethylprosta-5,9,13-trienoic acid, or its methyl or ethyl ester, (5Z,9Z,13E)-(15S)-11-oxo-15-hydroxy-15-(3-propylcyclopentyl)-16,17,18,19,20-pentanorprosta-5,9,13-trienoic acid, or its methyl or ethyl ester, (5Z,9Z,14EZ)-11-oxoprosta-5,9,12,14-tetraenoic acid, or its methyl or ethyl ester, (9Z,14EZ)-11-oxoprosta-9,12,14-trienoic acid, or its methyl or ethyl ester, (5Z,9Z,14EZ)-(17S)-11-oxo-17,20-dimethylprosta-5,9,12,14-tetraenoic acid, or its methyl or ethyl ester, (5Z,9Z,14EZ)-11-oxo-16,16-dimethylprosta-5,9,12,14-tetraenoic acid, or its methyl or ethyl ester, (5Z,9Z,14EZ)-11-oxo-15-(3-propylcyclopentyl)-16,17,18,19,20-pentanorprosta-5,9,12,14-tetraenoic acid, or its methyl or ethyl ester, (5Z,9Z,14EZ)-11-oxo-16-phenyl-17,18,19,20-tetranorprosta-5,9,12,14-tetraenoic acid, or its methyl or ethyl ester, or (5Z,9Z,14EZ)-11-oxo-16-(3-chlorophenoxy)-17,18,19,20-tetranorprosta-5,9,12,14-tetraenoic acid, or its methyl or ethyl ester.

6. A cyclodextrin clathrate of a prostaglandin derivative claimed in claim 5 or a non-toxic salt of a prostaglandin derivative acid as claimed in claim 5.

7. A process for the preparation of prostaglandin D derivatives as claimed in claim 1, which comprises, when the prostaglandin derivative conforms to the general formula (I) wherein the various symbols are as defined in claim 1,

(i) the hydrolysis under acidic conditions of a compound of the general formula:

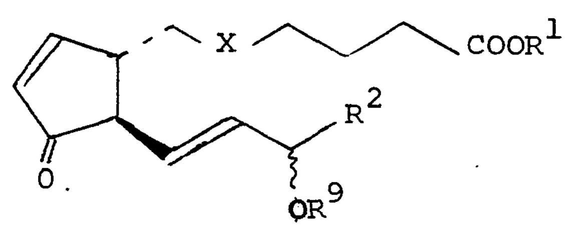

(VIa)

wherein $R^9$ represents a tetrahydropyran-2-yl group, a tetrahydrofuran-2-yl group either unsubstituted or substituted by at least one alkyl group, or a 1-ethoxyethyl group, and the other symbols are as defined in claim 1, or (ii) dehydration of a compound of the general formula:

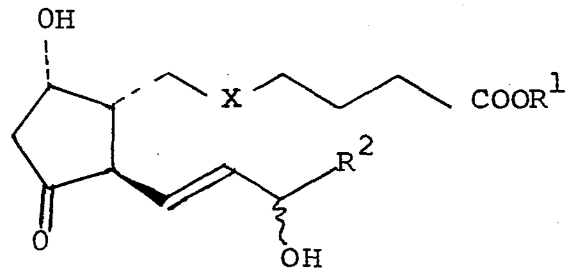

(IIIa)

(wherein the various symbols are as defined in claim 1) under mild conditions or, when the prostaglandin D derivative conforms to the general formula (II) wherein the various symbols are as defined in claim 1,

(iii) the reaction of a compound of the general formula:

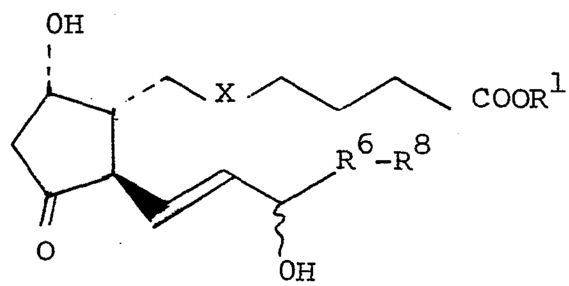

(III)

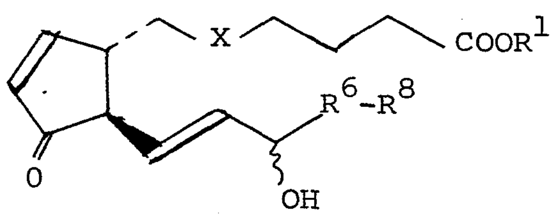

(IV)

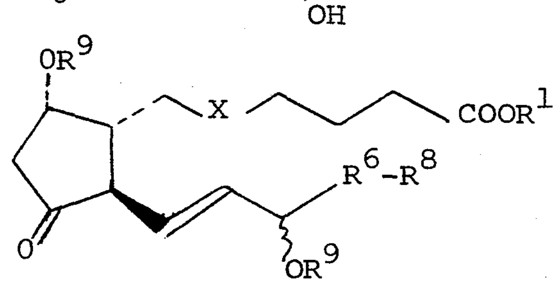

(V)

or

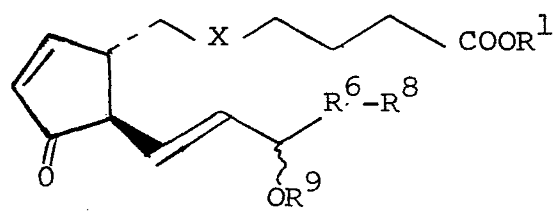

(VI)

wherein $R^9$ is as hereinbefore defined and the other symbols are as defined in claim 1, with an aqueous acid solution, optionally followed by the step of converting a prostaglandin derivative of general formula (I) or (II) wherein $R^1$ represents a hydrogen atom into an alkyl ester having from 1 to 12 carbon atoms in the alkyl radical or into a non-toxic salt, or by the step of converting a prostaglandin derivative of general formula (I) or (II) into a cyclodextrin clathrate thereof.

8. A pharmaceutical composition which comprises, as active ingredient at least one prostaglandin derivative of the general formula (I) or (II) depicted in claim 1, wherein the various symbols are as defined in claim 1, or a cyclodextrin clathrate thereof, or, when $R^1$ in formula (I) or (II) depicted in claim 1 represents a hydrogen atom, a non-toxic salt thereof, in association with a pharmaceutical carrier or coating.

9. A compound of the general formula (VIa), (V) or (VI) depicted in claim 7 wherein $R^9$ is as defined in claim 7 and the other symbols are as defined in claim 1.

10. A prostaglandin derivative or cyclodextrin clathrate thereof or non-toxic salt thereof, as claimed in claim 1, for use in the prevention or therapy of leukemia or solid cancer.

CLAIMS

CONTRACTING STATE: AT

1. A process for the preparation of a prostaglandin D derivative of the general formula:

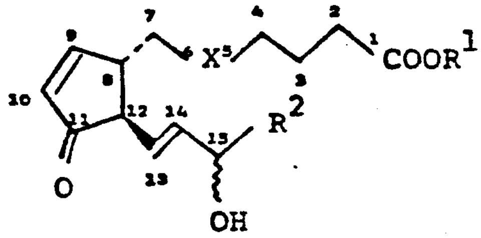

(I)

wherein $R^1$ represents a hydrogen atom or a straight-chain or branched-chain alkyl group of 1 - 12 carbon atoms, $R^2$ represents a group of the general formula:

(i) 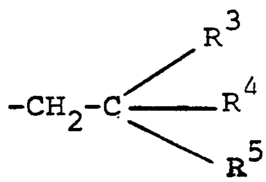 or

(ii) $-R^6-R^7$

wherein $R^3$ represents a straight-chain or branched-chain alkyl group of 1 - 4 carbon atoms, $R^4$ represents a hydrogen atom or a straight-chain or branched-chain alkyl group of 1 - 4 carbon atoms, $R^5$ represents a straight-chain or branched-chain alkyl group of 1 - 10 carbon atoms, $R^6$ represents a single bond or a straight-chain or branched-chain alkylene group of 1 - 5 carbon atoms, and $R^7$ represents a cycloalkyl group of 4 - 7 carbon atoms either unsubstituted or substituted by at least one straight-chain or branched-chain alkyl group of 1 - 8 carbon atoms,

X represents an ethylene group or a cis-vinylene group, the double bond between $C_9 - C_{10}$ is Z, and the double bond between $C_{13} - C_{14}$ is E, or of the general formula:

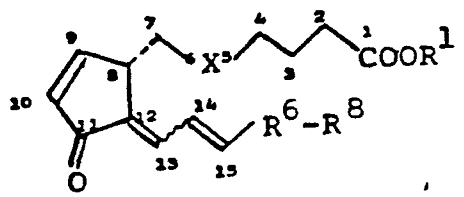

(II)

wherein $R^1$, $R^6$ and X are as hereinbefore defined, $R^8$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms or represents a phenyl group or phenoxy group either unsubstituted or substituted by at least one halogen atom, trifluoromethyl group or straight-chain or branched-chain alkyl group of 1 - 4 carbon atoms, the double bonds between $C_{12} - C_{13}$ and between $C_{14} - C_{15}$, which may be in the same or different configuration, are E, Z or a mixture thereof, and the double bond between $C_9 - C_{10}$ is Z, provided that when $R^6$ represents a single bond, $R^8$ does not represent a substituted or unsubstituted phenoxy group, or cyclodextrin clathrate of a compound of formula (I) or (II) or, when $R^1$ in the general formula (I) or (II) represents a hydrogen atom, a non-toxic salt thereof, which comprises, when the prostaglandin

derivative conforms to general formula (I), wherein the various symbols are as hereinbefore defined:

(i) the hydrolysis under acidic conditions of a compound of the general formula:

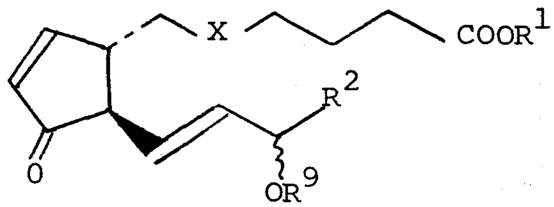

(VIa)

wherein $R^9$ represents a tetrahydropyran-2-yl group, a tetrahydrofuran-2-yl group either unsubstituted or substituted by at least one alkyl group, or a 1-ethoxyethyl group, and the other symbols are as hereinbefore defined, or (ii) dehydration of a compound of the general formula:

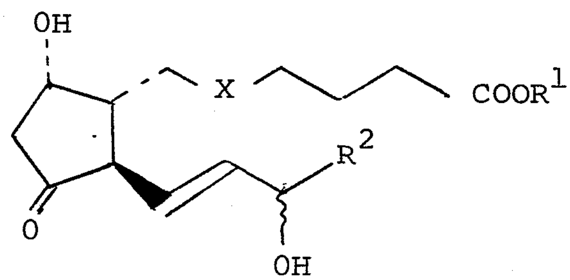

(IIIa)

(wherein the various symbols are as hereinbefore defined) under mild conditions; or, when the prostaglandin derivative conforms to the general formula (II), wherein the various symbols are as hereinbefore defined,

(iii) the reaction of a compound of the general formula:

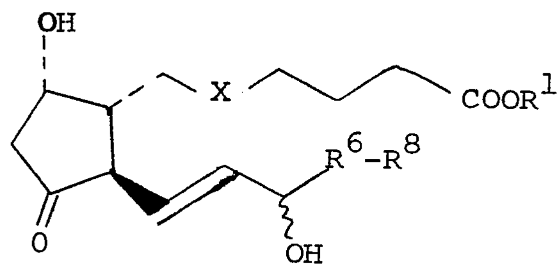

(III)

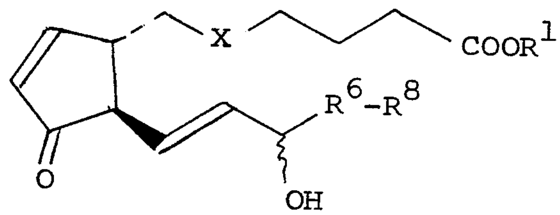

(IV)

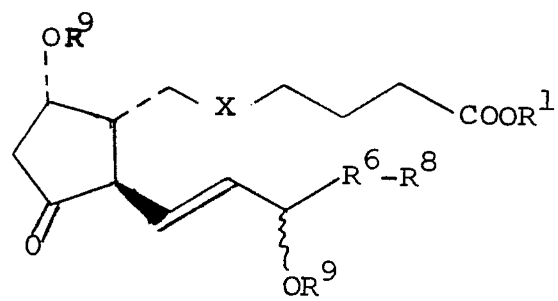

(V)

or

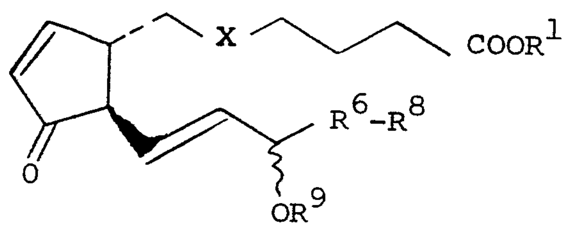

(VI)

(wherein the various symbols are as hereinbefore defined) with an aqueous acid solution followed, if desired, (a) by the step of converting a prostaglandin derivative of general formula (I) or (II) wherein $R^1$ represents a hydrogen atom into an alkyl ester having from 1 to 12 carbon atoms in the alkyl radical or into a non-toxic salt, or (b) by the step of converting a prostaglandin derivative of general formula (I) or (II) into a cyclodextrin clathrate thereof.

2. Process according to claim 1 wherein the symbol $R^9$ in the compound of general formula (VIa) represents a tetrahydropyran-2-yl group and the other symbols are as defined in claim 1.

3. A process according to claim 1 or 2, wherein the hydrolysis under acidic conditions of the compound of the general formula (VIa), wherein the various symbols are as defined in claim 1 or 2, is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulphonic acid and anhydrous methanol.

4. A process according to claim 1, wherein the dehydration under mild conditions of a compound of the general formula IIIa, wherein the various symbols are

as defined in claim 1, is carried out by reaction with a tris hydrochloric acid buffer solution at a temperature of 30-40°C.

5. A process according to claim 1, wherein the reaction of a compound of the general formula (III), (IV), (V) or (VI), wherein the various symbols are as defined in claim 1, with an aqueous acid solution is carried out in tetrahydrofuran using 1N hydrochloric acid at the reflux temperature of the reaction mixture.